(19)

Europäisches
Patentamt

European
Patent Office

Office européen
des brevets

(11) **EP 3 967 754 A1**

(12) **EUROPEAN PATENT APPLICATION**
published in accordance with Art. 153(4) EPC

(43) Date of publication:
**16.03.2022 Bulletin 2022/11**

(21) Application number: **20805937.8**

(22) Date of filing: **08.05.2020**

(51) International Patent Classification (IPC):
*C12N 9/06* (2006.01)          *A61K 38/44* (2006.01)
*A61K 47/56* (2017.01)          *A61P 19/02* (2006.01)
*A61P 19/06* (2006.01)          *A61P 13/12* (2006.01)

(52) Cooperative Patent Classification (CPC):
**A61K 38/44; A61K 47/56; A61P 3/00; A61P 3/06;
A61P 3/10; A61P 9/10; A61P 9/12; A61P 13/12;
A61P 19/02; A61P 19/06; A61P 29/00; A61P 35/00**

(86) International application number:
**PCT/CN2020/089272**

(87) International publication number:
**WO 2020/228618 (19.11.2020 Gazette 2020/47)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **10.05.2019 CN 201910388717**

(71) Applicants:
• **Peg-Bio Biopharm Co., Ltd. (Chongqing)**
**Chongqing 400714 (CN)**
• **Hangzhou Grand Biologic Pharmaceutical Inc.**
**Hangzhou, Zhejiang 310019 (CN)**

(72) Inventors:
• **FAN, Kai**
**Chongqing 400714 (CN)**
• **WANG, Zhiming**
**Hangzhou, Zhejiang 310019 (CN)**
• **LIU, Riyong**
**Chongqing 400714 (CN)**
• **WANG, Yu**
**Hangzhou, Zhejiang 310019 (CN)**
• **HE, Yunfeng**
**Chongqing 400714 (CN)**

• **YAN, Tianwen**
**Hangzhou, Zhejiang 310019 (CN)**
• **FU, Zhicheng**
**Chongqing 400714 (CN)**
• **SU, Guowei**
**Hangzhou, Zhejiang 310019 (CN)**
• **HU, Chunlan**
**Chongqing 400714 (CN)**
• **DING, Xupeng**
**Hangzhou, Zhejiang 310019 (CN)**
• **TAN, Changcheng**
**Chongqing 400714 (CN)**
• **WANG, Hongying**
**Hangzhou, Zhejiang 310019 (CN)**
• **YANG, Hui**
**Chongqing 400714 (CN)**
• **DING, Qiong**
**Chongqing 400714 (CN)**
• **WEN, Haiyan**
**Chongqing 400714 (CN)**

(74) Representative: **Habermann, Hruschka &
Schnabel
Patentanwälte
Montgelasstraße 2
81679 München (DE)**

(54) **POLYETHYLENE GLYCOL-MODIFIED URATE OXIDASE**

(57)    The present disclosure provides a polyethylene glycol-modified urate oxidase. At least 11 of the following amino acid sites in the urate oxidase have a PEG modification: $T^1$, $K^3$, $K^4$, $K^{30}$, $K^{35}$, $K^{76}$, $K^{79}$, $K^{97}$, $K^{112}$, $K^{116}$, $K^{120}$, $K^{152}$, $K^{179}$, $K^{222}$, $K^{231}$, $K^{266}$, $K^{272}$, $K^{285}$, $K^{291}$, and $K^{293}$.

SEQUENCE LISTING

<110>  HANGZHOU GRAND BIOLOGIC PHARMACEUTICAL INC.
       PEG-BIO BIOPHARM CO., LTD. (CHONGQING)

<120> POLYETHYLENE GLYCOL-MODIFIED URATE OXIDASE

<130>  PIDC3196280P

<160>  7

<170>  PatentIn version 3.3

<210>  1
<211>  298
<212>  PRT
<213>  Artificial

<220>
<223>  Amino acid sequence of chimeric uricase (pig-baboon) derived from pig
and baboon

<400>  1

Thr Tyr Lys Lys Asn Asp Glu Val Glu Phe Val Arg Thr Gly Tyr Gly
1               5                   10                  15

Lys Asp Met Ile Lys Val Leu His Ile Gln Arg Asp Gly Lys Tyr His
            20                  25                  30

Ser Ile Lys Glu Val Ala Thr Thr Val Gln Leu Thr Leu Ser Ser Lys
            35                  40                  45

Lys Asp Tyr Leu His Gly Asp Asn Ser Asp Val Ile Pro Thr Asp Thr
        50                  55                  60

Ile Lys Asn Thr Val Asn Val Leu Ala Lys Phe Lys Gly Ile Lys Ser
65                  70                  75                  80

Ile Glu Thr Phe Ala Val Thr Ile Cys Glu His Phe Leu Ser Ser Phe
                85                  90                  95

Lys His Val Ile Arg Ala Gln Val Tyr Val Glu Glu Val Pro Trp Lys
                100                 105                 110

Arg Phe Glu Lys Asn Gly Val Lys His Val His Ala Phe Ile Tyr Thr
            115                 120                 125

Pro Thr Gly Thr His Phe Cys Glu Val Glu Gln Ile Arg Asn Gly Pro
        130                 135                 140

Pro Val Ile His Ser Gly Ile Lys Asp Leu Lys Val Leu Lys Thr Thr
145                 150                 155                 160

```
Gln Ser Gly Phe Glu Gly Phe Ile Lys Asp Gln Phe Thr Thr Leu Pro
                165                 170                 175

Glu Val Lys Asp Arg Cys Phe Ala Thr Gln Val Tyr Cys Lys Trp Arg
                180                 185                 190

Tyr His Gln Gly Arg Asp Val Asp Phe Glu Ala Thr Trp Asp Thr Val
                195                 200                 205

Arg Ser Ile Val Leu Gln Lys Phe Ala Gly Pro Tyr Asp Lys Gly Glu
    210                 215                 220

Tyr Ser Pro Ser Val Gln Lys Thr Leu Tyr Asp Ile Gln Val Leu Thr
225                 230                 235                 240

Leu Gly Gln Val Pro Glu Ile Glu Asp Met Glu Ile Ser Leu Pro Asn
                245                 250                 255

Ile His Tyr Leu Asn Ile Asp Met Ser Lys Met Gly Leu Ile Asn Lys
                260                 265                 270

Glu Glu Val Leu Leu Pro Leu Asp Asn Pro Tyr Gly Lys Ile Thr Gly
                275                 280                 285

Thr Val Lys Arg Lys Leu Ser Ser Arg Leu
    290                 295
```

```
<210>   2
<211>   304
<212>   PRT
<213>   Artificial

<220>
<223>   Amino acid sequence of pig-derived urate oxidase

<400>   2
```

```
Met Ala His Tyr Arg Asn Asp Tyr Lys Lys Asn Asp Glu Val Glu Phe
1               5                   10                  15

Val Arg Thr Gly Tyr Gly Lys Asp Met Ile Lys Val Leu His Ile Gln
                20                  25                  30

Arg Asp Gly Lys Tyr His Ser Ile Lys Glu Val Ala Thr Ser Val Gln
                35                  40                  45

Leu Thr Leu Ser Ser Lys Lys Asp Tyr Leu His Gly Asp Asn Ser Asp
    50                  55                  60
```

```
Val Ile Pro Thr Asp Thr Ile Lys Asn Thr Val Asn Val Leu Ala Lys
65              70              75              80

Phe Lys Gly Ile Lys Ser Ile Glu Thr Phe Ala Val Thr Ile Cys Glu
                85              90              95

His Phe Leu Ser Ser Phe Lys His Val Ile Arg Ala Gln Val Tyr Val
            100             105             110

Glu Glu Val Pro Trp Lys Arg Phe Glu Lys Asn Gly Val Lys His Val
            115             120             125

His Ala Phe Ile Tyr Thr Pro Thr Gly Thr His Phe Cys Glu Val Glu
        130             135             140

Gln Ile Arg Asn Gly Pro Pro Val Ile His Ser Gly Ile Lys Asp Leu
145             150             155             160

Lys Val Leu Lys Thr Thr Gln Ser Gly Phe Glu Gly Phe Ile Lys Asp
            165             170             175

Gln Phe Thr Thr Leu Pro Glu Val Lys Asp Arg Cys Phe Ala Thr Gln
            180             185             190

Val Tyr Cys Lys Trp Arg Tyr His Gln Gly Arg Asp Val Asp Phe Glu
        195             200             205

Ala Thr Trp Asp Thr Val Arg Ser Ile Val Leu Gln Lys Phe Ala Gly
        210             215             220

Pro Tyr Asp Lys Gly Glu Tyr Ser Pro Ser Val Gln Lys Thr Leu Tyr
225             230             235             240

Asp Ile Gln Val Leu Thr Leu Gly Gln Val Pro Glu Ile Glu Asp Met
            245             250             255

Glu Ile Ser Leu Pro Asn Ile His Tyr Leu Asn Ile Asp Met Ser Lys
            260             265             270

Met Gly Leu Ile Asn Lys Glu Glu Val Leu Leu Pro Leu Asp Asn Pro
        275             280             285

Tyr Gly Arg Ile Thr Gly Thr Val Lys Arg Lys Leu Thr Ser Arg Leu
        290             295             300
```

```
<210>    3
<211>    297
<212>    PRT
```

EP 3 967 754 A1

<213> Artificial

<220>
<223> Amino acid sequence of chimeric urate oxidase (canine-baboon) derived from canine and baboon

<400> 3

```
Met Tyr Lys Asn Asp Glu Val Glu Phe Val Arg Thr Gly Tyr Gly Lys
1               5                   10                  15

Asp Met Val Lys Val Leu His Ile Gln Arg Asp Gly Lys Tyr His Ser
            20                  25                  30

Ile Lys Glu Val Ala Thr Ser Val Gln Leu Thr Leu Ser Ser Lys Lys
        35                  40                  45

Asp Tyr Val Tyr Gly Asp Asn Ser Asp Ile Ile Pro Thr Asp Thr Ile
    50                  55                  60

Lys Asn Thr Val His Val Leu Ala Lys Phe Lys Gly Ile Lys Ser Ile
65              70                  75                  80

Glu Thr Phe Ala Met Asn Ile Cys Glu His Phe Leu Ser Ser Phe Asn
                85                  90                  95

His Val Ile Arg Ala Gln Val Tyr Val Glu Glu Val Pro Trp Lys Arg
            100                 105                 110

Phe Glu Lys Asn Gly Val Lys His Val His Ala Phe Ile His Asn Pro
        115                 120                 125

Thr Gly Thr His Phe Cys Glu Val Glu Gln Met Arg Ser Gly Pro Pro
    130                 135                 140

Val Ile His Ser Gly Ile Lys Asp Leu Lys Val Leu Lys Thr Thr Gln
145                 150                 155                 160

Ser Gly Phe Glu Gly Phe Ile Lys Asp Gln Phe Thr Thr Leu Pro Glu
                165                 170                 175

Val Lys Asp Arg Cys Phe Ala Thr Lys Val Tyr Cys Lys Trp Arg Tyr
            180                 185                 190

His Gln Gly Arg Asp Val Asp Phe Glu Ala Thr Trp Asp Thr Val Arg
        195                 200                 205

Asp Ile Val Leu Glu Lys Phe Ala Gly Pro Tyr Asp Lys Gly Glu Tyr
    210                 215                 220
```

41

Ser Pro Ser Val Gln Lys Thr Leu Tyr Asp Ile Gln Val His Ser Leu
225                 230                 235                 240

Ser Arg Val Pro Glu Met Glu Asp Met Glu Ile Ser Leu Pro Asn Ile
                245                 250                 255

His Tyr Phe Asn Ile Asp Met Ser Lys Met Gly Leu Ile Asn Lys Glu
                260                 265                 270

Glu Val Leu Leu Pro Leu Asp Asn Pro Tyr Gly Lys Ile Thr Gly Thr
                275                 280                 285

Val Lys Arg Lys Leu Ser Ser Arg Leu
                290                 295


<210>   4
<211>   304
<212>   PRT
<213>   Artificial

<220>
<223>   Amino acid sequence of canine-derived urate oxidase

<400>   4

Met Ala His Tyr His Asn Asp Tyr Lys Lys Asn Asp Glu Val Glu Phe
1                   5                   10                  15

Val Arg Thr Gly Tyr Gly Lys Asp Met Val Lys Val Leu His Ile Gln
                20                  25                  30

Arg Asp Gly Lys Tyr His Ser Ile Lys Glu Val Ala Thr Ser Val Gln
                35                  40                  45

Leu Thr Leu Ser Ser Lys Lys Asp Tyr Val Tyr Gly Asp Asn Ser Asp
                50                  55                  60

Ile Ile Pro Thr Asp Thr Ile Lys Asn Thr Val His Val Leu Ala Lys
65                  70                  75                  80

Phe Lys Gly Ile Lys Ser Ile Glu Thr Phe Ala Met Asn Ile Cys Glu
                85                  90                  95

His Phe Leu Ser Ser Phe Asn His Val Ile Arg Ala Gln Val Tyr Val
                100                 105                 110

Glu Glu Val Pro Trp Lys Arg Phe Glu Lys Asn Gly Val Lys His Val
                115                 120                 125

```
His Ala Phe Ile His Asn Pro Thr Gly Thr His Phe Cys Glu Val Glu
    130                 135                 140

Gln Met Arg Ser Gly Pro Pro Val Ile His Ser Gly Ile Lys Asp Leu
    145                 150                 155                 160

Lys Val Leu Lys Thr Thr Gln Ser Gly Phe Glu Gly Phe Ile Lys Asp
                165                 170                 175

Gln Phe Thr Thr Leu Pro Glu Val Lys Asp Arg Cys Phe Ala Thr Lys
                180                 185                 190

Val Tyr Cys Lys Trp Arg Tyr His Gln Gly Arg Asp Val Asp Phe Glu
                195                 200                 205

Ala Thr Trp Asp Thr Val Arg Asp Ile Val Leu Glu Lys Phe Ala Gly
    210                 215                 220

Pro Tyr Asp Lys Gly Glu Tyr Ser Pro Ser Val Gln Lys Thr Leu Tyr
225                 230                 235                 240

Asp Ile Gln Val His Ser Leu Ser Arg Val Pro Glu Met Glu Asp Met
                245                 250                 255

Glu Ile Ser Leu Pro Asn Ile His Tyr Phe Asn Ile Asp Met Ser Lys
                260                 265                 270

Met Gly Leu Ile Asn Lys Glu Glu Val Leu Leu Pro Leu Asp Asn Pro
    275                 280                 285

Tyr Gly Arg Ile Thr Gly Thr Ala Lys Arg Lys Leu Ala Ser Lys Leu
    290                 295                 300


<210>  5
<211>  304
<212>  PRT
<213>  Artificial

<220>
<223>  Amino acid sequence of bovine-derived urate oxidase

<400>  5

Met Ala His Tyr His Asn Asp Tyr Gln Lys Asn Asp Glu Val Glu Phe
1               5                   10                  15

Val Arg Thr Gly Tyr Gly Lys Asp Met Val Lys Val Leu His Ile Gln
            20                  25                  30

Arg Asp Gly Lys Tyr His Ser Ile Lys Glu Val Ala Thr Ser Val Gln
```

|  | 35 |  | 40 |  | 45 |  |
|---|---|---|---|---|---|---|

Leu Thr Leu Asn Ser Arg Arg Glu Tyr Leu His Gly Asp Asn Ser Asp
    50            55           60

Ile Ile Pro Thr Asp Thr Ile Lys Asn Thr Val Gln Val Leu Ala Lys
65            70          75           80

Phe Lys Gly Ile Lys Ser Ile Glu Thr Phe Ala Met Asn Ile Cys Glu
          85          90          95

His Phe Leu Ser Ser Phe Asn His Val Ile Arg Val Gln Val Tyr Val
       100        105        110

Glu Glu Val Pro Trp Lys Arg Phe Glu Lys Asn Gly Val Lys His Val
      115        120        125

His Ala Phe Ile His Thr Pro Thr Gly Thr His Phe Cys Glu Val Glu
    130           135        140

Gln Leu Arg Ser Gly Pro Pro Val Ile His Ser Gly Ile Lys Asp Leu
145            150         155          160

Lys Val Leu Lys Thr Thr Gln Ser Gly Phe Glu Gly Phe Leu Lys Asp
       165        170        175

Gln Phe Thr Thr Leu Pro Glu Val Lys Asp Arg Cys Phe Ala Thr Gln
       180        185        190

Val Tyr Cys Lys Trp Arg Tyr His Gln Gly Arg Asp Val Asp Phe Glu
      195        200        205

Ala Thr Trp Glu Ala Val Arg Gly Ile Val Leu Lys Lys Phe Ala Gly
    210           215        220

Pro Tyr Asp Lys Gly Glu Tyr Ser Pro Ser Val Gln Lys Thr Leu Tyr
225            230         235          240

Asp Ile Gln Val Leu Ser Leu Ser Gln Leu Pro Glu Ile Glu Asp Met
       245        250        255

Glu Ile Ser Leu Pro Asn Ile His Tyr Phe Asn Ile Asp Met Ser Lys
       260        265        270

Met Gly Leu Ile Asn Lys Glu Glu Val Leu Leu Pro Leu Asp Asn Pro
       275        280        285

Tyr Gly Arg Ile Thr Gly Thr Val Lys Arg Lys Leu Thr Ser Arg Leu
    290             295             300

<210>  6
<211>  304
<212>  PRT
<213>  Artificial

<220>
<223>  Amino acid sequence of monkey-derived urate oxidase

<400>  6

Met Ala His Tyr His Asn Asp Tyr Lys Lys Asn Asp Glu Val Glu Phe
1               5               10              15

Val Arg Thr Gly Tyr Gly Lys Asp Met Val Lys Val Leu His Ile Gln
            20              25              30

Arg Asp Gly Lys Tyr His Ser Ile Lys Glu Val Ala Thr Ser Val Gln
            35              40              45

Leu Thr Leu Ser Ser Lys Lys Asp Tyr Leu His Gly Asp Asn Ser Asp
    50              55              60

Ile Ile Pro Thr Asp Thr Ile Lys Asn Thr Val His Ala Leu Ala Lys
65              70              75              80

Phe Lys Gly Ile Lys Ser Ile Glu Ala Phe Ala Val Asn Ile Cys Gln
            85              90              95

His Phe Leu Ser Ser Phe Asn His Val Ile Arg Thr Gln Val Tyr Val
            100             105             110

Glu Glu Ile Pro Trp Lys Arg Leu Glu Lys Asn Gly Val Lys His Val
            115             120             125

His Ala Phe Ile His Thr Pro Thr Gly Thr His Phe Cys Glu Val Glu
    130             135             140

Gln Leu Arg Ser Gly Pro Pro Val Ile His Ser Gly Ile Lys Asp Leu
145             150             155             160

Lys Val Leu Lys Thr Thr Gln Ser Gly Phe Glu Gly Phe Ile Lys Asp
            165             170             175

Gln Phe Thr Thr Leu Pro Glu Val Lys Asp Arg Cys Phe Ala Ala Gln
            180             185             190

Val Tyr Cys Lys Trp Arg Tyr His Gln Cys Arg Asp Val Asp Phe Glu

```
              195                    200                    205

       Ala Thr Trp Asp Thr Ile Arg Asp Val Val Leu Glu Lys Phe Ala Gly
           210                215                220

       Pro Tyr Asp Lys Gly Glu Tyr Ser Pro Ser Val Gln Lys Thr Leu Tyr
       225                230                235                240

       Asp Ile Gln Val Val Ser Leu Ser Gln Val Pro Glu Ile Asp Asp Met
                       245                250                255

       Glu Ile Ser Leu Pro Asn Ile His Tyr Phe Asn Ile Asp Met Ser Lys
                   260                265                270

       Met Gly Leu Ile Asn Lys Glu Glu Val Leu Leu Pro Leu Asp Asn Pro
                   275                280                285

       Tyr Gly Lys Ile Thr Gly Thr Val Lys Arg Lys Leu Ser Ser Arg Leu
           290                295                300


       <210>  7
       <211>  304
       <212>  PRT
       <213>  Artificial

       <220>
       <223>  Amino acid sequence of baboon-derived urate oxidase

       <400>  7

       Met Ala Asp Tyr His Asn Asn Tyr Lys Lys Asn Asp Glu Leu Glu Phe
       1               5                10                15

       Val Arg Thr Gly Tyr Gly Lys Asp Met Val Lys Val Leu His Ile Gln
                   20                25                30

       Arg Asp Gly Lys Tyr His Ser Ile Lys Glu Val Ala Thr Ser Val Gln
                   35                40                45

       Leu Thr Leu Ser Ser Lys Lys Asp Tyr Leu His Gly Asp Asn Ser Asp
               50                55                60

       Ile Ile Pro Thr Asp Thr Ile Lys Asn Thr Val His Val Leu Ala Lys
       65                70                75                80

       Phe Lys Gly Ile Lys Ser Ile Glu Ala Phe Gly Val Asn Ile Cys Glu
                       85                90                95

       Tyr Phe Leu Ser Ser Phe Asn His Val Ile Arg Ala Gln Val Tyr Val
                   100                105                110
```

```
Glu Glu Ile Pro Trp Lys Arg Leu Glu Lys Asn Gly Val Lys His Val
        115             120             125

His Ala Phe Ile His Thr Pro Thr Gly Thr His Phe Cys Glu Val Glu
        130             135             140

Gln Leu Arg Ser Gly Pro Pro Val Ile His Ser Gly Ile Lys Asp Leu
145             150             155                     160

Lys Val Leu Lys Thr Thr Gln Ser Gly Phe Glu Gly Phe Ile Lys Asp
                165             170                     175

Gln Phe Thr Thr Leu Pro Glu Val Lys Asp Arg Cys Phe Ala Thr Gln
            180             185             190

Val Tyr Cys Lys Trp Arg Tyr His Gln Cys Arg Asp Val Asp Phe Glu
        195             200             205

Ala Thr Trp Gly Thr Ile Arg Asp Leu Val Leu Glu Lys Phe Ala Gly
    210             215             220

Pro Tyr Asp Lys Gly Glu Tyr Ser Pro Ser Val Gln Lys Thr Leu Tyr
225             230             235                     240

Asp Ile Gln Val Leu Ser Leu Ser Arg Val Pro Glu Ile Glu Asp Met
            245             250             255

Glu Ile Ser Leu Pro Asn Ile His Tyr Phe Asn Ile Asp Met Ser Lys
            260             265             270

Met Gly Leu Ile Asn Lys Glu Glu Val Leu Leu Pro Leu Asp Asn Pro
        275             280             285

Tyr Gly Lys Ile Thr Gly Thr Val Lys Arg Lys Leu Ser Ser Arg Leu
    290             295             300
```

**Description**

**FIELD**

The present disclosure relates to the field of biomedicine. Specifically, the present disclosure relates to a polyethylene glycol-modified urate oxidase, and more specifically, the present disclosure relates to a polyethylene glycol-modified urate oxidase, a pharmaceutical composition, pharmaceutical uses of the polyethylene glycol-modified urate oxidase, and a method for reducing immunogenicity of urate oxidase.

**BACKGROUND**

Gout is a disease caused by the disorder of purine metabolism, with a clinical feature of hyperuricemia, and tophus is formed due to the deposition of urate under the skin, at joints, and in kidneys. The purine in the human body undergoes a series of changes, and the formed final product is uric acid. Hyperuricemia may occur when a concentration of uric acid in blood exceeds 70 mg/L. Among them, 5% to 12% of patients with hyperuricemia may progress to gout. Once a concentration of sodium urate in blood or synovial fluid reaches a saturated state, microcrystals of sodium urate salt can be formed, inducing gouty arthritis. Over time, chronic hyperuricemia may also cause the deposition of destructive crystalline uric acid deposits around joints, in soft tissues, and in certain organs, thereby inducing diseases such as acute gouty arthritis and chronic tophus arthritis, and joint deformities. Kidney damage is considered to be the second most common clinical manifestation of gout. The progressive nature of chronic hyperuricemia causes urate deposition in the medulla, renal tubules, and renal interstitium, stimulating the local area and causing inflammation, which is called chronic urate nephropathy. For patients with severe hyperuricemia (such as patients with certain malignant tumors, especially leukemia and lymphoma), a large amount of uric acid may be deposited in the renal collecting duct, renal pelvis, calyx and ureter in a short period of time, resulting in obstruction of lumen and anuresis and inducing acute renal failure (also known as uric acid nephropathy).

In recent decades, with the improvement of people's living quality and changes in diet and living habits, the intake of high-protein and high-purine foods has increased, and the number of gout patients has been increasing year by year. In Europe, the number of gout patients has approximately doubled in the past 20 years. The current incidence of hyperuricemia and gout in China has increased to about 2% to 3%. If the clinical symptoms of hyperuricemia do not appear, it is only needed to control the diet. When the clinical symptoms caused by hyperuricemia appear, drug treatment is required. Conventional clinical treatments currently include: analgesic and anti-inflammatory drugs, such as colchicine, ibuprofen, naproxen, etc., which are mainly used to control the symptoms of acute attacks of gouty arthritis, eliminating local pain, swelling and inflammation of the joints; uricosuric agents that promote the excretion of uric acid (ineffective if the kidney function is reduced), such as probenicid, sulfinpyrazone, benzbromarone, etc.; drugs that inhibit the synthesis of uric acid, such as allopurinol. Allopurinol is the main therapeutic drug for patients suffering from tophus gout, renal insufficiency, leukemia, and certain genetic diseases, and it can inhibit xanthine oxidase to disenable the conversion from hypoxanthine and xanthine into uric acid. Allopurinol can be gradually oxidized in vivo to produce oxipurinol, which is easily soluble in water and excreted with the urine. However, the patients with chronic gout and with the formed tophus can be hardly cured by either of these conventional treatments. In addition, after long-term use of the above-mentioned drugs, patients will inevitably experience complications such as leukopenia, impaired heart function, impaired liver and kidney function, stimulation to the gastrointestinal system, aplastic anemia leading to diabetes, or gout.

Human hyperuricemia is related to the mutation and inactivation of the uricase gene during the evolution process. The mutation introduces premature termination codons into the coding sequence of the human uricase gene (Wu X, Lee C C, Muzny D M, Caskey C T. Proc Natl Acad Sci USA. 1989.86: 9412-9416.). Therefore, human beings themselves cannot synthesize active uricase, so that human purine catabolism is terminated at uric acid (Wu X, Muzny D M, Lee C, Caskey C T. J Mol Evol. 1992. 34: 78-84.). The active uricase in the liver peroxisomes of non-human primates and other mammals can convert the less soluble urate (about 11 mg/100 ml water) into the more soluble allantoin (about 147 mg/100 ml water), which can be excreted more effectively by the kidneys (Wortmann R L, Kelley W N. Kelley's textbook of rheumatology (6th). 2001: 1339-1376). In Europe and America, uricase (Uricozyme) prepared by Aspergillus flavus has been used in the treatment of severe hyperuricemia associated with tumor chemotherapy for more than 10 years (Zittoun R, Dauchy F, Teilaud C, Barthelemy M, Bouchard P. Ann Med Interne. 1978.127: 479-482.).The recombinant Aspergillus flavus uricase drug, ELITEK, developed by the Sanofi (France) and produced by fermentation of beer yeast, has been approved by the FDA in 2002 and used for the short-term treatment of severe hyperuricemia induced by tumor chemotherapy (Pui C H, Relling M V, Lascombes F, HarrisonP L, Struxiano A et al.Leukemia.1997.11: 1813-1816.). Meanwhile, it was proved that the infusion of ELITEK can also reduce the volume of tophus (Potaux L, Aparicio M, Maurel C, Ruedas M E, Mart in C L. Nouv PresseMed. 1975.4: 1109-1112.). The PEG-modified recombinant porcine uricase (Pegloticase), which was approved by the FDA in September 2010 and produced by Savient (USA), is used for the treatment of refractory gout, but it was invalid in clinical applications for 50% of patients due to its failure in solving the problem of immunogenicity.

Uricase (E C 1.7.3.3) is widely present in microorganisms (Bacillus fastidious, Candida monocytogenes, Aspergillus flavus), plants (soybeans, chickpeas), animals (pigs, cattle, dogs, baboons) (Suzuki K, Sakasegawa S, Misaki H, Sugiyama M. J Biosci Bioeng. 2004. 98: 153-158), and in the presence of oxygen, it can catalyze uric acid to oxidize allantoin to release carbon dioxide (Retailleau P, Colloc'h, Denis V, Francoise B. Acta Cryst D.2004.60: 453-462.).

The active uricase is a tetrameric protein, composed of the same subunits, and each subunit has a molecular weight of about 34kD and is composed of 301 to 304 amino acids. The pH at the highest enzyme activity of uricase in each solution is 8.0 (Bayol A etal.Biophys Chem.1995.54: 229-235.). Among all the sources of uricase currently known, the uricase with the highest activity is from Aspergillus flavus, up to 27 IU/mg; and the second is from Bacillus fastidious, with an activity maintained at 13 IU/mg (Huang S H, Wu T K. Eur J Biochem. 2004. 271: 517-523.). In addition, the uricase derived from legumes only has an activity of 2 to 6 IU/mg; for the uricase derived from mammals, after recombinant expression, the activity of the uricase derived from pigs can reach 5 IU/mg, the activity of the uricase derived from baboons is only 1 IU/mg (Michael H, Susan J.K. 2006. US7056713B1), and the uricase derived from human beings is inactive.

For the application in human body, due to the high activity of microbial uricase and the low immunogenicity of mammalian uricase, the uricase derived from these two sources became a research focus in the current development and application of recombinant uricase. However, the homology between the uricase derived from Aspergillus flavus and the predicted human uricase is less than 40% (Lee C C, Wu X, Gibbs R A, Cook R G, Muzny D M, CaskeyC T.Science.1988.239: 1288-1291.), the human body is prone to produce antibodies against the uricase, which results in a rapid weakening of the effect of Aspergillus flavus uricase and at the same time causes severe allergic reactions, and thus Aspergillus flavus uricase cannot be used for long-term treatment.

Therefore, the technology for the treatment of hyperuricemia based on urate oxidase still needs further development and improvement.

## SUMMARY

The present disclosure is based on the Applicant's findings and knowledge of the following facts and problems:

The active urate oxidase is a homotetrameric protein, one-third of the amino acids of which are strongly hydrophobic amino acids, and the tetrameric proteins may easily aggregate to form octamers and larger aggregates. Molecules with a molecular weight above 100 kDa can effectively induce the body to produce an immune response, the molecular weight of the unmodified polymer urate oxidase protein has already reached 140 kDa, and the polymer uricase with greater molecular weight will have higher immunogenicity. The human body is prone to produce antibodies against uricase, thereby rapidly weakening the efficacy thereof and causing severe allergic reactions, and thus the uricase is unsuitable for long-term treatment. It has been proven that through covalent modification of proteins with PEG, the protein immunogenicity can be reduced, the protein solubility can be increased, and the protein half-life can be prolonged.

Duke University and Savient conducted research on chimeric uricase derived from pigs and baboons (Michael H, Susan J.K. 2006. US7056713B1). In this research, ε-amino group of lysine residue of the urease derived from pig-like sources was modified with methoxy-containing polyethylene glycol having a molecular weight of 10 kDa (10 kDa-mPEG-NPC), the modified product is Pegloticase, and thus the goal of treating intractable gout in the human body has been initially achieved without significantly reducing enzyme activity. Applicant found that the above-mentioned research results cannot completely solve the immunogenicity problem caused by the drug. Clinical subjects have experienced the disappearance of the uricase efficacy after multiple injections, which, according to Applicant's speculations, may be related to the excessively great molecular weight of the Pegloticase protein (using 10kd of PEG leads to Pegloticase having a molecular weight of 540 kDa). Further, since pegloticase is not suitable for injection, but suitable for intravenous bolus injection, the subjects' compliance with long-term use is reduced, thereby severely limiting its clinical application. So far, there is no long-acting urate oxidase drug having lower immunogenicity and suitable for subcutaneous injection.

The present disclosure aims to at least solve one of the technical problems in the related art to a certain extent.

In a first aspect of the present disclosure, the present disclosure provides a polyethylene glycol-modified urate oxidase. According to embodiments of the present disclosure, at least 11 of the following amino acid sites in the urate oxidase have a PEG modification: $T^1$, $K^3$, $K^4$, $K^{30}$, $K^{35}$, $K^{76}$, $K^{79}$, $K^{97}$, $K^{112}$, $K^{116}$, $K^{120}$, $K^{152}$, $K^{179}$, $K^{222}$, $K^{231}$, $K^{266}$, $K^{272}$, $K^{285}$, $K^{291}$, and $K^{293}$. It should be noted that the "urate oxidase" mentioned in the present disclosure should be understood in a broad sense, and it refers to the general name of a mixture of urate oxidases produced in one same batch in actual production practice. Applicant found that, compared with analogous drugs already on the market, at least 11 of the above-mentioned amino acid sites in the polyethylene glycol-modified urate oxidase of the embodiments of the present disclosure have the PEG modification, which can significantly improve the in vivo stability of urate oxidase while guaranteeing the maximum enzyme activity, reduces the immunogenicity is reduced, and has an in vivo efficacy after intramuscular injection equivalent to an in vivo efficacy after intravenous injection of analogous drugs on the market.

According to the embodiments of the present disclosure, the urate oxidase may further include at least one of the following additional technical features.

According to an embodiment of the present disclosure, at least one, at least two, at least three, or four of the following 4 amino acid sites in the urate oxidase have the PEG modification: $K^{30}$, $K^{35}$, $K^{222}$, and $K^{231}$.

According to an embodiment of the present disclosure, polyethylene glycol used for the PEG modification has a molecular weight smaller than or equal to 6KD. Applicant found that, using the polyethylene glycol with a molecular weight smaller than or equal to 6KD for modification, the obtained urate oxidase has further enhanced long-lasting efficacy in vivo, and will not produce serious anti-PEG antibodies due to the excessive molecular weight, that is, the immunogenicity is further reduced .

According to an embodiment of the present disclosure, the polyethylene glycol has a monomethoxyl group or a hydroxyl group.

According to an embodiment of the present disclosure, the polyethylene glycol is a linear or branched structure.

According to an embodiment of the present disclosure, the polyethylene glycol and the urate oxidase are coupled through an amide bond.

According to an embodiment of the present disclosure, the polyethylene glycol is a modifying polyethylene glycol, and a modifying group of the modifying polyethylene glycol includes at least one selected from the group consisting of: N-hydroxysuccinimide, a N-hydroxysuccinimidyl carbonate ester, a N-hydroxysuccinimidyl acetate ester, a N-hydroxysuccinimidyl propionate ester, a N-hydroxysuccinimidyl butyrate ester, a N-hydroxysuccinyl succinate ester, and a bis(4-nitrophenyl) carbonate ester.

According to an embodiment of the present disclosure, the modifying group of the modifying polyethylene glycol is the N-hydroxysuccinimidyl propionate ester.

According to an embodiment of the present disclosure, the amino acid sites are positioned based on an amino acid sequence set forth as SEQ ID NO: 1.

TYKKNDEVEFVRTGYGKDMIKVLHIQRDGKYHSIKEVATTVQLTLSSKKD YLHGDNSDVIPTDTIKNTVNVLAKFKGIKSIETFAVTICEHFLSSFKHVIRAQVYVEEVP WKRFEKNGVKHVHAFIYTPTGTHFCEVEQIRNGPPVIHSGIKDLKVLKTTQSGFEGFI KDQFTTLPEVKDRCFATQVYCKWRYHQGRDVDFEATWDTVRSIVLQKFAGPYDKGE YSPSVQKTLYDIQVLTLGQVPEIEDMEISLPNIHYLNIDMSKMGLINKEEVLLPLDNPY GKITGTVKRKLSSRL (SEQ ID NO: 1).

According to an embodiment of the present disclosure, the urate oxidase has amino acid sequences set forth as SEQ ID NO: 1 to SEQ ID NO: 7; or the urate oxidase has a polypeptide having at least 70%, at least 75%, at least 80%, at least 85%, at least 90%, at least 95%, or at least 99% identity to SEQ ID NO: 1 to SEQ ID NO: 7; or the urate oxidase has a polypeptide having the amino acid sequences set forth as SEQ ID NO: 1 to SEQ ID NO: 7 in which one or more amino acids are substituted, deleted and/or added.

     MAHYRNDYKKNDEVEFVRTGYGKDMIKVLHIQRDGKYHSIKEVATSVQL TLSSKKDYLHGDNSDVIPTDTIKNTVNVLAKFKGIKSIETFAVTICEHFLSSFKHVIRAQ VYVEEVPWKRFEKNGVKHVHAFIYTPTGTHFCEVEQIRNGPPVIHSGIKDLKVLKTTQ SGFEGFIKDQFTTLPEVKDRCFATQVYCKWRYHQGRDVDFEATWDTVRSIVLQKFAG PYDKGEYSPSVQKTLYDIQVLTLGQVPEIEDMEISLPNIHYLNIDMSKMGLINKEEVLL PLDNPYGRITGTVKRKLTSRL (SEQ ID NO: 2).

     MYKNDEVEFVRTGYGKDMVKVLHIQRDGKYHSIKEVATSVQLTLSSKKD YVYGDNSDIIPTDTIKNTVHVLAKFKGIKSIETFAMNICEHFLSSFNHVIRAQVYVEEV PWKRFEKNGVKHVHAFIHNPTGTHFCEVEQMRSGPPVIHSGIKDLKVLKTTQSGFEG FIKDQFTTLPEVKDRCFATKVYCKWRYHQGRDVDFEATWDTVRDIVLEKFAGPYDK GEYSPSVQKTLYDIQVHSLSRVPEMEDMEISLPNIHYFNIDMSKMGLINKEEVLLPLDN PYGKITGTVKRKLSSRL (SEQ ID NO: 3).

MAHYHNDYKKNDEVEFVRTGYGKDMVKVLHIQRDGKYHSIKEVATSVQL
TLSSKKDYVYGDNSDIIPTDTIKNTVHVLAKFKGIKSIETFAMNICEHFLSSFNHVIRAQ
VYVEEVPWKRFEKNGVKHVHAFIHNPTGTHFCEVEQMRSGPPVIHSGIKDLKVLKTT
QSGFEGFIKDQFTTLPEVKDRCFATKVYCKWRYHQGRDVDFEATWDTVRDIVLEKFA
GPYDKGEYSPSVQKTLYDIQVHSLSRVPEMEDMEISLPNIHYFNIDMSKMGLINKEEV
LLPLDNPYGRITGTAKRKLASKL (SEQ ID NO: 4).


MAHYHNDYQKNDEVEFVRTGYGKDMVKVLHIQRDGKYHSIKEVATSVQL
TLNSRREYLHGDNSDIIPTDTIKNTVQVLAKFKGIKSIETFAMNICEHFLSSFNHVIRVQ
VYVEEVPWKRFEKNGVKHVHAFIHTPTGTHFCEVEQLRSGPPVIHSGIKDLKVLKTT
QSGFEGFLKDQFTTLPEVKDRCFATQVYCKWRYHQGRDVDFEATWEAVRGIVLKKFA
GPYDKGEYSPSVQKTLYDIQVLSLSQLPEIEDMEISLPNIHYFNIDMSKMGLINKEEVL
LPLDNPYGRITGTVKRKLTSRL (SEQ ID NO: 5).


MAHYHNDYKKNDEVEFVRTGYGKDMVKVLHIQRDGKYHSIKEVATSVQL
TLSSKKDYLHGDNSDIIPTDTIKNTVHALAKFKGIKSIEAFAVNICQHFLSSFNHVIRTQ
VYVEEIPWKRLEKNGVKHVHAFIHTPTGTHFCEVEQLRSGPPVIHSGIKDLKVLKTTQ
SGFEGFIKDQFTTLPEVKDRCFAAQVYCKWRYHQCRDVDFEATWDTIRDVVLEKFAG


PYDKGEYSPSVQKTLYDIQVVSLSQVPEIDDMEISLPNIHYFNIDMSKMGLINKEEVLL
PLDNPYGKITGTVKRKLSSRL (SEQ ID NO: 6).


MADYHNNYKKNDELEFVRTGYGKDMVKVLHIQRDGKYHSIKEVATSVQL
TLSSKKDYLHGDNSDIIPTDTIKNTVHVLAKFKGIKSIEAFGVNICEYFLSSFNHVIRAQ
VYVEEIPWKRLEKNGVKHVHAFIHTPTGTHFCEVEQLRSGPPVIHSGIKDLKVLKTTQ
SGFEGFIKDQFTTLPEVKDRCFATQVYCKWRYHQCRDVDFEATWGTIRDLVLEKFAG
PYDKGEYSPSVQKTLYDIQVLSLSRVPEIEDMEISLPNIHYFNIDMSKMGLINKEEVLLP
LDNPYGKITGTVKRKLSSRL (SEQ ID NO: 7).

The amino acid sequence set forth as SEQ ID NO: 1 is an amino acid sequence of a chimeric uricase (pig-baboon) derived from pig and baboon; the amino acid sequence set forth as SEQ ID NO: 2 is an amino acid sequence of pig-derived urate oxidase; the amino acid sequence set forth as SEQ ID NO: 3 is an amino acid sequence of a chimeric uricase (canine-baboon) derived from canine and baboon; the amino acid sequence set forth as SEQ ID NO: 4 is an amino acid sequence of canine-derived urate oxidase; the amino acid sequence set forth as SEQ ID NO: 5 is an amino acid sequence of bovine-derived urate oxidase; the amino acid sequence set forth as SEQ ID NO: 6 is an amino acid sequence of monkey-derived urate oxidase; and the amino acid sequence set forth as SEQ ID NO: 7 is an amino acid sequence of baboon-derived urate oxidase.

It should be noted that lysine in the present disclosure is positioned based on the amino acid sequence set forth as SEQ ID NO: 1. For example, $K^4$ refers to the lysine located at position 4 based on the amino acid sequence set forth as SEQ ID NO: 1. The uricase has the amino acid sequences set forth as SEQ ID NO: 1 to SEQ ID NO: 7, or the polypeptide having at least 70%, at least 75%, at least 80%, at least 85%, at least 90%, at least 95%, or at least 99% identity to SEQ ID NO: 1 to SEQ ID NO: 7, or the polypeptide having the amino acid sequences set forth as SEQ ID NO: 1 to SEQ ID NO: 7 in which one or more amino acids are substituted, deleted and/or added have homology in their structures. Those skilled in the art, through sequence mapping, can determine respective positions corresponding to $T^1$, $K^3$, $K^4$, $K^{30}$, $K^{35}$, $K^{76}$, $K^{79}$, $K^{97}$, $K^{112}$, $K^{116}$, $K^{120}$, $K^{152}$, $K^{179}$, $K^{222}$, $K^{231}$, $K^{266}$, $K^{272}$, $K^{285}$, $K^{291}$, and $K^{293}$ on SEQ ID NO: 2 to SEQ ID NO: 7, or the polypeptide having at least 70%, at least 75%, at least 80%, at least 85%, at least 90%, at least 95%, or at least 99% identity to SEQ ID NO: 2 to SEQ ID NO: 7, or the polypeptide having the amino acid sequences set forth as SEQ ID NO: 1 to SEQ ID NO: 7 in which one or more amino acids are substituted, deleted and/or added, and then allow the PEG modification to occur at the corresponding positions on the above-mentioned polypeptide sequences, thereby achieving the advantages of the polyethylene glycol-modified urate oxidase of the present disclosure, such as low immunogenicity, high stability in vivo, and applicability for intramuscular injection. The sequence alignment method is a common method used by those skilled in the art to perform sequence identity comparison of isozymes with different sequence sources. Different sequences may have differences in sequence length due to mutations, deletions, etc., but those skilled in the art can determine the identity between different sequences by means of sequence alignment.

For example, according to the embodiments of the present disclosure, sites of the sequence set forth as SEQ ID NO: 2 corresponding to sites $T^1$, $K^3$, $K^4$, $K^{30}$, $K^{35}$, $K^{76}$, $K^{79}$, $K^{97}$, $K^{112}$, $K^{116}$, $K^{120}$, $K^{152}$, $K^{179}$, $K^{222}$, $K^{231}$, $K^{266}$, $K^{272}$, $K^{285}$, $K^{291}$, , and $K^{293}$ of the sequence set forth as SEQ ID NO: 1 include $M^1$, $K^9$, $K^{10}$, $K^{36}$, $K^{41}$, $K^{82}$, $K^{85}$, $K^{103}$, $K^{118}$, $K^{122}$, $K^{126}$, $K^{158}$, $K^{185}$, $K^{228}$, $K^{237}$, $K^{272}$, $K^{278}$, $K^{297}$, and $K^{299}$; sites of the sequence set forth as SEQ ID NO: 3 corresponding to the above-mentioned respective sites of the sequence set forth as SEQ ID NO: 1 include $M^1$, $K^3$, $K^{29}$, $K^{34}$, $K^{75}$, $K^{78}$, $K^{111}$, $K^{115}$, $K^{119}$, $K^{151}$, $K^{178}$, $K^{221}$, $K^{230}$, $K^{265}$, $K^{271}$, $K^{284}$, $K^{290}$, and $K^{292}$; sites of the sequence set forth as SEQ ID NO: 4 corresponding to the above-mentioned respective sites of the sequence set forth as SEQ ID NO: 1 include $M^1$, $K^9$, $K^{10}$, $K^{36}$, $K^{41}$, $K^{82}$, $K^{85}$, $K^{118}$, $K^{122}$, $K^{126}$, $K^{158}$, $K^{185}$, $K^{228}$, $K^{237}$, $K^{272}$, $K^{278}$, $K^{297}$, and $K^{299}$; sites of the sequence set forth as SEQ ID NO: 5 corresponding to the above-mentioned respective sites of the sequence set forth as SEQ ID NO: 1 include $M^1$, $K^{10}$, $K^{36}$, $K^{41}$, $K^{82}$, $K^{85}$, $K^{118}$, $K^{122}$, $K^{126}$, $K^{158}$, $K^{185}$, $K^{228}$, $K^{237}$, $K^{272}$, $K^{278}$, $K^{297}$, and $K^{299}$; sites of the sequence set forth as SEQ ID NO: 6 corresponding to the above-mentioned respective sites of the sequence set forth as SEQ ID NO: 1 include $M^1$, $K^9$, $K^{10}$, $K^{36}$, $K^{41}$, $K^{82}$, $K^{85}$, $K^{118}$, $K^{122}$, $K^{126}$, $K^{158}$, $K^{185}$, $K^{228}$, $K^{237}$, $K^{272}$, $K^{278}$, $K^{291}$, $K^{297}$, and $K^{299}$; sites of the sequence set forth as SEQ ID NO: 7 corresponding to the above-mentioned respective sites of the sequence set forth as SEQ ID NO: 1 include $M^1$, $K^9$, $K^{10}$, $K^{36}$, $K^{41}$, $K^{82}$, $K^{85}$, $K^{118}$, $K^{122}$, $K^{126}$, $K^{158}$, $K^{185}$, $K^{228}$, $K^{237}$, $K^{272}$, $K^{278}$, $K^{291}$, $K^{297}$, and $K^{299}$. Applicant found through experiments that, after at least 11 sites of the respective sites of the amino acid sequence set forth as SEQ ID NO: 2 to SEQ ID NO: 7 are modified with PEG, the obtained PEG-modified urate oxidase has low immunogenicity, high stability in vivo, and applicability for intramuscular injection.

In a second aspect of the present disclosure, the present disclosure provide a polyethylene glycol-modified urate oxidase. According to an embodiment of the present disclosure, peak areas of at least 11 predetermined peptide fragments in a peptide map of the polyethylene glycol-modified urate oxidase are reduced by a relative proportion of 75% or more, preferably 80% or more, more preferably 90% or more, compared with those in a peptide map of urate oxidase unmodified with polyethylene glycol. The polyethylene glycol-modified urate oxidase according to the embodiment of the present disclosure has the advantages of low immunogenicity, high stability in vivo, and applicability for intramuscular injection.

According to the embodiments of the present disclosure, the polyethylene glycol-modified urate oxidase may further include at least one of the following additional technical features.

According to an embodiment of the present disclosure, the peptide map of the polyethylene glycol-modified urate oxidase has peptide fragments with peak area reduction shown in Table 5.

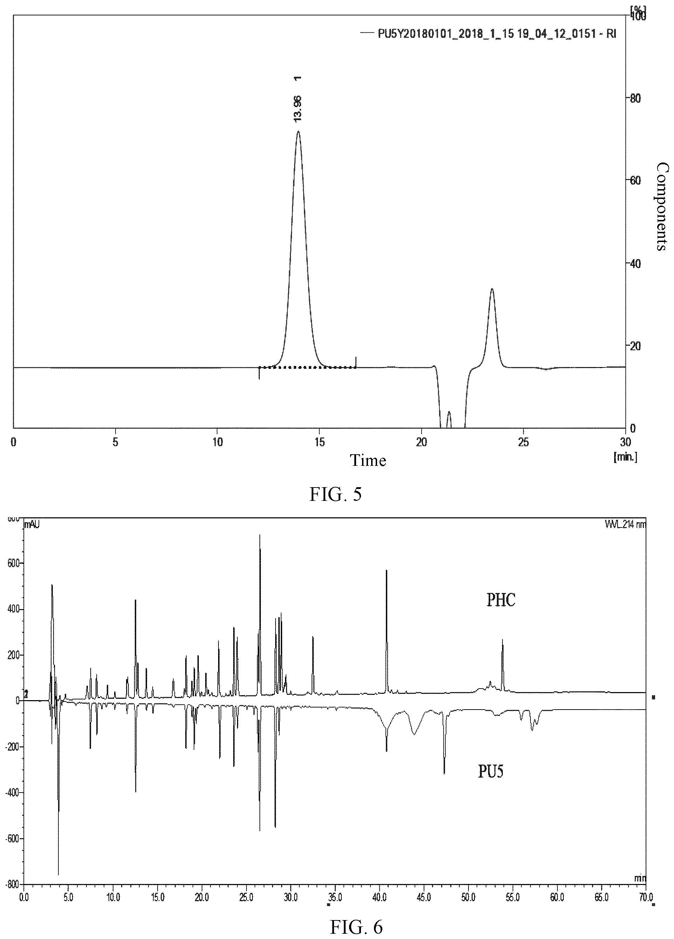

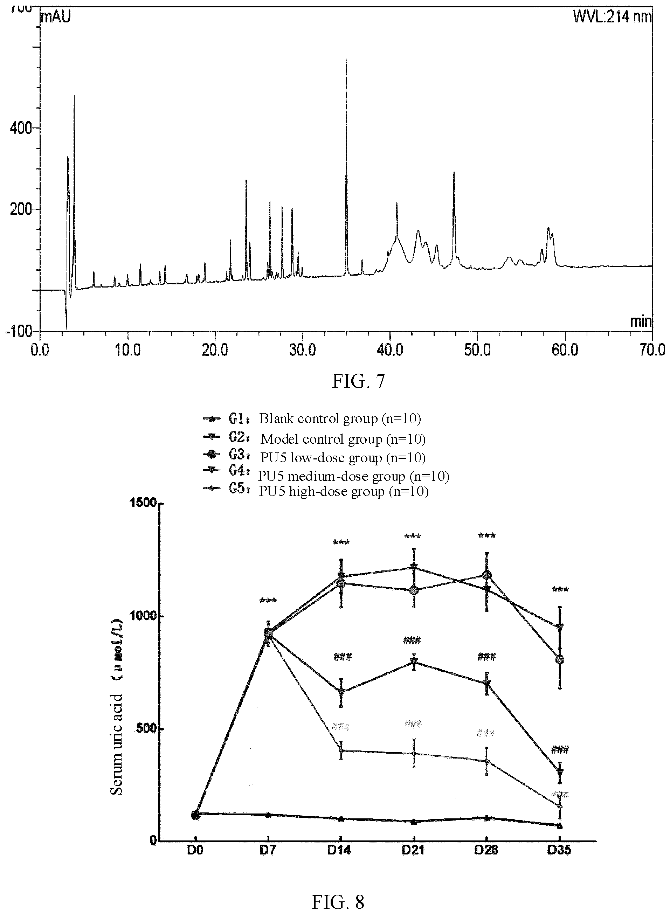

According to an embodiment of the present disclosure, the peptide map of the polyethylene glycol-modified urate oxidase is shown in FIG. 6 or FIG. 7.

According to a third aspect of the present disclosure, the present disclosure provides a pharmaceutical composition. According to the embodiments of the present disclosure, the pharmaceutical composition includes the above-mentioned polyethylene glycol-modified urate oxidase. The pharmaceutical composition according to the embodiments of the present disclosure has the advantages of low immunogenicity, high stability in vivo, and applicability for intramuscular injection and can be used for the treatment or prevention of hyperuric acid-related diseases.

According to the embodiments of the present disclosure, the pharmaceutical composition further includes at least one of the following additional technical features.

According to an embodiment of the present disclosure, the pharmaceutical composition further includes an additional drug for treatment or prevention of hyperuric acid-related diseases.

In a fourth aspect of the present disclosure, the present disclosure provides use of the urate oxidase as described above or the pharmaceutical composition as described above in manufacture of a medicament for treating hyperuric acid-related diseases and reducing a uric acid level in a biological fluid of a subject in need thereof. The urate oxidase according to the embodiments of the present disclosure has the advantages such as low immunogenicity, high stability in vivo, and applicability for intramuscular injection, and has significant advantages in the treatment of the hyperuric acid-related diseases.

According to the embodiments of the present disclosure, the above use may include at least one of the following additional technical features.

According to embodiments of the present disclosure, the hyperuric acid-related diseases include chronic hyperuricemia, gout, kidney disease, hyperuricemic arthritis, renal calculi, tophus, hypertension, diabetes, hypertriglyceridemia, mtabolic syndrome, coronary heart disease, atherosclerosis, and cancer chemotherapy-induced hyperuricemia.

According to an embodiment of the present disclosure, the biological fluid is urine or blood.

In a fifth aspect of the present disclosure, the present disclosure provides a method for reducing immunogenicity of urate oxidase. According to the embodiments of the present disclosure, the method includes: enabling at least 11 of the following amino acid sites in the urate oxidase to have a PEG modification: $T^1$, $K^3$, $K^4$, $K^{30}$, $K^{35}$, $K^{76}$, $K^{79}$, $K^{97}$, $K^{112}$, $K^{116}$, $K^{120}$, $K^{152}$, $K^{179}$, $K^{222}$, $K^{231}$, $K^{266}$, $K^{272}$, $K^{285}$, $K^{291}$, and $K^{293}$. The method according to the embodiments of the present disclosure can effectively reduce the immunogenicity of urate oxidase, and the obtained urate oxidase is safer and long lasting in vivo.

According to the embodiments of the present disclosure, the method may further include at least one of the following additional technical features.

According to an embodiment of the present disclosure, the method includes enabling at least one, at least two, at least three, or four of the following 4 amino acid sites in the urate oxidase to have the PEG modification: $K^{30}$, $K^{35}$, $K^{222}$, and $K^{231}$.

According to an embodiment of the present disclosure, polyethylene glycol used for the PEG modification has a molecular weight smaller than or equal to 6KD.

According to an embodiment of the present disclosure, the polyethylene glycol is a modifying polyethylene glycol.

According to an embodiment of the present disclosure, a modifying group of the modifying polyethylene glycol is N-hydroxysuccinimide.

According to an embodiment of the present disclosure, the amino acid sites are positioned based on the amino acid sequence set forth as SEQ ID NO: 1.

According to the embodiments of the present disclosure, the urate oxidase has amino acid sequences set forth as SEQ ID NO: 1 to SEQ ID NO: 7; or the urate oxidase has a polypeptide having at least 70%, at least 75%, at least 80%, at least 85%, at least 90%, at least 95%, or at least 99% identity to SEQ ID NO: 1 to SEQ ID NO: 7; or the urate oxidase has a polypeptide having the amino acid sequences set forth as SEQ ID NO: 1 to SEQ ID NO: 7 in which one or more amino acids are substituted, deleted and/or added.

It can be understood that the technical effects of the aforementioned additional technical features of the polyethylene glycol-modified urate oxidase are applicable to the additional technical features of the aforementioned method for reducing the immunogenicity of urate oxidase according to the embodiments of the present disclosure. Thus, the technical effects of the aforementioned additional technical features of the aforementioned method for reducing the immunogenicity of urate oxidase according to the embodiments of the present disclosure will not be repeated herein.

**BRIEF DESCRIPTION OF DRAWINGS**

FIG. 1 is a spectrum of SEC-HPLC-UV detection of a PHC physical and chemical reference substance according to an embodiment of the present disclosure;

FIG. 2 is a spectrum of SEC-HPLC-RI detection of a PHC physical and chemical reference substance according to an embodiment of the present disclosure;

FIG. 3 is a spectrum of SEC-HPLC-RI detection of a PEG reference substance according to an embodiment of the present disclosure;

FIG. 4 is a spectrum of SEC-HPLC-UV detection of a PU5 modification product according to an embodiment of the present disclosure;

FIG. 5 is a spectrum of SEC-HPLC-RI detection of a PU5 modification product according to an embodiment of the present disclosure;

FIG. 6 is a comparison diagram of PHC and PU5 using Lys-c and trypsin double digestion according to an embodiment of the present disclosure;

FIG. 7 is a diagram of Lys-C digestion of PU5 according to an embodiment of the present disclosure;

FIG. 8 illustrates serum uric acid levels after intramuscular administration of different doses to model rats according to an embodiment of the present disclosure;

FIG. 9 is a diagram illustrating scores of kidney injury, necrosis, and inflammation according to an embodiment of the present disclosure;

FIG. 10 is a graph illustrating mean serum drug concentration-time curve of various groups after a single intravenous injection of the same dose (1.0 mg/kg) of Pegloticase and a pegylated uricase injection in SD rats according to an embodiment of the present disclosure;

FIG. 11 illustrates mean serum drug concentration-time curves of various groups after a single intramuscular injection of Pegloticase and pegylated uricase injections of different doses in SD rats according to an embodiment of the present disclosure;

FIG. 12 illustrates mean serum drug concentration-time curves of various groups after a single intramuscular injection of pegylated uricase injections of different doses in SD rats according to an embodiment of the present disclosure;

FIG. 13 illustrates mean serum uric acid value-time curves of various groups after a single intramuscular/intravenous injection of Pegloticase and pegylated uricase injections of different doses in SD rats according to an embodiment of the present disclosure;

FIG. 14 illustrates mean serum drug concentration-time curves of male and female SD rats after the first intravenous injection (Day 1) of the same dose (1.0 mg/kg) of Pegloticase and a pegylated uricase injection according to an embodiment of the present disclosure;

FIG. 15 illustrates mean serum drug concentration-time curves of male and female SD rats after the last intravenous injection (Day 22) of the same dose (1.0 mg/kg) of Pegloticase and a pegylated uricase injection according to an embodiment of the present disclosure;

FIG. 16 illustrates mean serum drug concentration-time curves of male and female SD rats after the first intramuscular injection (Day 1) of the same dose (1.0 mg/kg) of Pegloticase and a pegylated uricase injection according to an embodiment of the present disclosure;

FIG. 17 illustrates mean serum drug concentration-time curves of male and female SD rats after the last intramuscular injection (Day 22) of the same dose (1.0 mg/kg) of Pegloticase and a pegylated uricase injection according to an embodiment of the present disclosure;

FIG. 18 illustrates mean serum uric acid value-time curves after multiple intravenous injections of Pegloticase and a pegylated uricase injection in SD rats according to an embodiment of the present disclosure; and

FIG. 19 illustrates mean serum uric acid value-time curves after multiple intramuscular injections of Pegloticase and a pegylated uricase injection in SD rats according to an embodiment of the present disclosure.

## DESCRIPTION OF EMBODIMENTS

The embodiments of the present disclosure are described in detail below, and examples of the embodiments are illustrated in the accompanying drawings. The embodiments described below with reference to the accompanying drawings are illustrative and are intended to explain the present disclosure, but should not be understood as a limitation of the present disclosure.

An object of the present disclosure is to provide a new type of polyethylene glycol-modified urate oxidase.

Another object of the present disclosure is to provide a method for effectively reducing immunogenicity of urate oxidase. This technology can effectively reduce the immunogenicity of urate oxidase and improve the in vivo safety and stability of urate oxidase.

Another object of the present disclosure is to provide uses of the polyethylene glycol-modified urate oxidase conjugate obtained above, which can achieve long-lasting efficacy in vivo and significantly reduce the serum uric acid level, and can be used for the treatment of hyperuricemia and gout.

In one aspect of the present disclosure, a polyethylene glycol-modified urate oxidase is provided.

As used herein, the terms the terms "urate oxidase" and "uricase" are interchangeable, and they both refer to a class of enzymes described in the present disclosure that can catalyze the oxidation of uric acid to produce allantoin and hydrogen peroxide. The terms "urate oxidase analogue", "uricase analogue", and "uricase derivative" are interchangeable, and they all refer to that parts of amino acids of a protein structural sequence of urate oxidase can be subjected to a structural modification such as substitution, deletion, or addition under the premise that the activity of urate oxidase for specifically catalyzing the conversion of uric acid into allantoin and hydrogen peroxide is maintained, thereby achieving the advantages of the present disclosure, including but not limited to reducing immunogenicity, increasing protein stability, and facilitating further polyethylene glycol modification.

Urate oxidase is not particularly limited, and can be urate oxidase and urate oxidase analogues derived from any source. Representative examples include, but are not limited to, mammalian sources, microorganisms, plants, etc.

In another preferred embodiment, the urate oxidase and urate oxidase analogues are derived from mammals, preferably, the amino acid sequences set forth as SEQ ID NO: 1, SEQ ID NO: 2, SEQ ID NO: 3, and SEQ ID NO: 4, and more preferably, the amino acid sequence set forth as SEQ ID NO: 1.

The urate oxidase derived from different species according to the present disclosure can be obtained through various manners, including but not limited to natural extraction, chemical synthesis, genetic engineering recombinant expression, etc.

In another preferred embodiment, through recombinant technology of urate oxidase, a coding sequence of the urate oxidase protein sequence (SEQ ID NO: 1) is subjected to recombinant expression in a host cell.

In another preferred embodiment, the urate oxidase is prepared and obtained in such a manner that *E. coli* or yeast is used as a host to construct a recombinant expression strain, and *E. coli* is more preferably used as host bacteria for recombinant expression.

As used herein, the polyethylene glycol-modified urate oxidase described in the present disclosure is obtained by covalently modifying urate oxidase with polyethylene glycol. The polyethylene glycol (PEG) refers to a mixture of ethylene oxide condensation polymer and water, represented by a general formula $H(OCH_2CH_2)_nOH$, which is a hy-

drophilic and linear or branched polymer with neutral pH and high water solubility and without toxicity. Due to the non-toxicity and good biocompatibility of PEG, the FDA has approved a variety of PEG-modified recombinant protein drugs on the market, proving that PEG can be used to reduce the immunogenicity of the protein, increase the solubility of the protein, and extend the half-life of the protein. In order to bind PEG to protein, one or more ends of PEG need to be activated, and the activation can be performed by selecting the corresponding modifying groups according to the groups on the target protein to be modified, such as amino, sulfhydryl, carboxyl or hydroxyl.

In another preferred embodiment, a site of oxidize uricase and uricase analogues for PEG modification according to the present disclosure is an ε-amino group of lysine residues, or an α-amino group of a small amount of N-terminal lysine residues. The urate oxidase is covalently connected to the modifying group of PEG through a urethane bond, a secondary amino bond, or an amide bond. Preferably, a polyethylene glycol molecule is coupled with urate oxidase to form an amide bond. The modifying groups of polyethylene glycol include, but are not limited to, N-hydroxysuccinimides, including but not limited to N-hydroxysuccinimide (NHS), N-hydroxysuccinimidyl carbonate (SC), N-hydroxysuccinimidyl acetate (SCM), N-hydroxysuccinimidyl propionate (SPA), N-hydroxysuccinimidyl butyrate (SBA), N-hydroxysuccinimidyl succinate (SS), etc. The blocking group of polyethylene glycol includes, but is not limited to, monomethoxy, ethoxy, glucose, or galactose, preferably monomethoxy.

In another preferred embodiment, polyethylene glycol may be linear or branched.

In another preferred embodiment, a relative molecular weight of the used polyethylene glycol for polyethylene glycol polyethylene glycol is not more than 6 KD, preferably 1 KD to 5 KD, more preferably 2 KD or 5 KD, and most preferably 5 KD. It should be noted that the "relative molecular weight of polyethylene glycol" mentioned herein refers to a relative molecular weight of polyethylene glycol without modifying groups, which has a general meaning in the related art. PEG, after the activation by the active groups, has a total relative molecular weight slightly greater than 5KD, for example, in a range of 5 KD + 10%.

In another preferred embodiment, the polyethylene glycol-modified urate oxidase has the following features:

(1) at least 11 amino acid sites of the following amino acid sites in the urate oxidase have PEG modification: $T^1$, $K^3$, $K^4$, $K^{30}$, $K^{35}$, $K^{76}$, $K^{79}$, $K^{97}$, $K^{112}$, $K^{116}$, $K^{120}$, $K^{152}$, $K^{179}$, $K^{222}$, $K^{231}$, $K^{266}$, $K^{272}$, $K^{285}$, $K^{291}$, , and $K^{293}$;
(2) one urate oxidase monomer molecule is coupled with 11 to 13 polyethylene glycol molecules on average;
(3) $K^{30}$ and/or $K^{35}$, as well as $K^{222}$ and/or $K^{231}$ contained in the urate oxidase sequence set forth as SEQ ID NO: 1 are modified and coupled with polyethylene glycol; and
(4) polyethylene glycol-modified urate oxidase has lower immunogenicity in vivo.

In another aspect of the present disclosure, a method for effectively reducing immunogenicity of urate oxidase is provided. This technology can effectively reduce the immunogenicity of urate oxidase and improve the in vivo stability of urate oxidase.

As used herein, the polyethylene glycol-modified urate oxidase is characterized in that, the urate oxidase is not particularly limited, and can be urate oxidase and urate oxidase analogues derived from any source, representative examples of which include, but are not limited to, sources of mammals, microorganisms, plants, etc..

In another preferred embodiment, the urate oxidase and the urate oxidase analogues are derived from mammals. The amino acid sequences of SEQ ID NO: 1, SEQ ID NO: 2, SEQ ID NO: 3, and SEQ ID NO: 4 are preferred, and the amino acid sequence of SEQ ID NO: 1 is more preferred.

The urate oxidase derived from different species according to the present disclosure can be obtained through various manners, including but not limited to natural extraction, chemical synthesis, genetic engineering recombinant expression, etc.

In another preferred embodiment, the urate oxidase is prepared and obtained in such a manner that *E. coli* or yeast is used as a host to construct a recombinant expression strain, and *E. coli* is more preferably used as host bacteria for recombinant expression.

The urate oxidase of the present disclosure is subjected to recombinant expression in *E. coli* to obtain a large amount of urate oxidase, and the expressed urate oxidase can be expressed in the cells, on the cell membranes, or secreted out of the cells. If necessary, methods known to those skilled in the art can be used to obtain high-purity urate oxidase. Examples of these methods include, but are not limited to, centrifugation, bacteria destruction, salting out, ultrafiltration, ion exchange chromatography, hydrophobic chromatography, molecular sieve chromatography, and a combination of various other techniques.

The urate oxidase obtained above can be covalently bound to polyethylene glycol through a linking group using methods known in the art.

In another preferred embodiment, polyethylene glycol directionally modifies the lysine residues on a surface of a spatial structure of urate oxidase. Urate oxidase is covalently connected to the modifying group (also referred to as active group) of PEG through an amide bond. The modifying groups (also referred to as active group) of polyethylene glycol include, but are not limited to, N-hydroxysuccinimide (NHS), N-hydroxysuccinimidyl carbonate (SC), N-hydroxysuccinimidyl acetate (SCM), N-hydroxysuccinimidyl propionate (SPA), N-hydroxysuccinimidyl butyrate (SBA), N-hydroxysuccinimidyl succinate (SS), etc. The blocking group of polyethylene glycol includes, but is not limited to, monomethoxy, ethoxy, glucose, or galactose, preferably monomethoxy.

In another preferred embodiment, polyethylene glycol may be linear or linear.

In another preferred embodiment, the relative molecular weight of the polyethylene glycol is not more than 6 KD, preferably 1 KD to 5 KD, and most preferably 5 KD.

In another preferred embodiment, the present disclosure provides a method for preparing the polyethylene glycol-modified urate oxidase. The method has one or more of the following features:

> (1) a modification feed molar ratio of urate oxidase to polyethylene glycol (urate oxidase: polyethylene glycol) ranges from 1:50 to 1:150, preferably from 1:55 to 1:95.
> (2) a coupling reaction system is carbonate buffer with a modification pH range ranging from 9 to 11.

The above-mentioned method for preparing the polyethylene glycol-modified urate oxidase can obtain high purity polyethylene glycol-modified urate oxidase by using a variety of purification methods.

In another preferred embodiment, the purification of the modified sample includes, but is not limited to, molecular sieve chromatography, ion exchange chromatography, hydrophobic chromatography, tangential flow ultrafiltration, or combinations thereof. More preferred are molecular sieve chromatography and tangential flow ultrafiltration.

In another aspect of the present disclosure, uses of the above-mentioned polyethylene glycol-modified urate oxidase are provided. The conjugate can achieve long-lasting efficacy in vivo and significantly reduce serum uric acid level, applicable for the treatment of hyperuricemia and gout.

The polyethylene glycol-modified urate oxidase is more suitable as a medicament and its composition for treating chronic hyperuricemia or gout. The main symptoms of hyperuricemia and gout include, but are not limited to, uric acid nephropathy and gouty arthritis.

The administration route of the polyethylene glycol-modified urate oxidase includes, but is not limited to, intravenous injection, subcutaneous injection, intramuscular injection, intraperitoneal injection, etc., preferably intravenous injection, intramuscular injection, and more preferably intramuscular injection.

The polyethylene glycol-modified urate oxidase has lower immunogenicity in vivo.

The low immunogenicity of the polyethylene glycol-modified urate oxidase means that, after intramuscular injection of the polyethylene glycol-modified urate oxidase in human body or animal body, the body does not produce antibodies against polyethylene glycol molecules or produces low titer antibodies against polyethylene glycol molecules, and also does not produce antibodies against urate oxidase.

The polyethylene glycol-modified urate oxidase has a longer half-life in vivo and the efficacy in reducing the uric acid level in the blood after intramuscular injection.

According to the embodiments of the present disclosure, under the premise of maintaining the enzyme activity to the maximal extent, the polyethylene glycol-modified urate oxidase of the present disclosure can greatly enhance the in vivo stability of urate oxidase and reduce the immunogenicity, and after intramuscular injection, the in vivo efficacy thereof can be equivalent to that of the marketed intravenous injection drugs. Therefore, the polyethylene glycol-modified urate oxidase of the present disclosure and a pharmaceutical composition containing the polyethylene glycol-modified urate oxidase can be administered when treating or preventing the hyperuric acid-related diseases.

The term "administration" as used herein refers to the introduction of a predetermined amount of a substance into a patient in a suitable manner. The polyethylene glycol-modified urate oxidase of the present disclosure can be administered through any common route, as long as it can reach the desired tissue. Various administration routes are conceivable, including peritoneal, intravenous, intramuscular, subcutaneous, cortical, oral, topical, nasal, pulmonary, and rectal administrations. The present disclosure is not limited to these illustrative administration routes. In addition, the pharmaceutical composition of the present disclosure can be administered using a specific device configured to deliver the active ingredient to the target cells.

The administration frequency and dose of the pharmaceutical composition of the present disclosure can be determined based on a number of related factors, including the type of disease to be treated, the administration route, the patient's age, gender, and weight, and the severity of the disease, as well as the type of the medicament serving as the active ingredient.

The term "therapeutically effective amount" refers to an amount of a compound that is sufficient to significantly ameliorate certain symptoms associated with a disease or condition, that is, an amount that provides a therapeutic effect for a given condition and dose regimen. For example, in the treatment of chronic hyperuricemia or gout, drugs or compounds that reduce, prevent, delay, inhibit, or block any symptoms of a disease or disorder should be therapeutically effective. A therapeutically effective amount of the drug or compound is not necessarily to cure the disease or condition, but will provide treatment for the disease or condition such that the onset of the disease or condition of an individual is delayed, blocked, or prevented, or the symptoms of the disease or condition are alleviated, or the duration of the disease or condition is changed, or, for example, the disease or illness becomes less serious, or recovery is accelerated.

The term "treatment" is used to refer to obtaining a desired pharmacological and/or physiological effect. Said effect may be prophylactic in terms of complete or partial prevention of the disease or its symptoms, and/or may be therapeutic in terms of partial or complete cure of the disease and/or adverse effects caused by the disease. The "treatment" as used herein covers the treatment of diseases of mammals, especially human (mainly referring to the hyperuric acid-related diseases), including: (a) prevention of diseases in individuals who are prone to a disease but have not yet been diagnosed; (b) inhibition of a disease, such as blocking the development of the disease; or (c) alleviation of a disease, such as reducing the symptoms associated with the disease. The "treatment" as used herein encompasses any medication that administers a medicament or compound to an individual to treat, cure, alleviate, ameliorate, reduce or inhibit the individual's disease, including but not limited to administering the polyethylene glycol-modified urate oxidase described herein to the individual in need thereof.

According to the embodiments of the present disclosure, the polyethylene glycol-modified urate oxidase or pharmaceutical composition of the present disclosure can be used in combination with conventional treatment methods and/or therapies, or can be used separately with conventional treatment methods and/or therapies. When the polyethylene glycol-modified urate oxidase or pharmaceutical composition of the present disclosure is administered in combination therapy with other drugs, they can be administered to the individual sequentially or simultaneously. Alternatively, the pharmaceutical composition of the present disclosure may include a combination of the polyethylene glycol-modified urate oxidase of the present disclosure, a pharmaceutically acceptable carrier or pharmaceutically acceptable excipient, and other therapeutic or preventive drugs known in the art.

The term "average modification degree" refers to the number of PEGs bound to each uricase monomer.

In the present disclosure, unless otherwise specified, the expression "amino acid site has PEG modification" means that, in a three-dimensional structure of a corresponding polypeptide, the PEG molecule covers the amino acid site in such a manner that at least a part of groups at the amino acid site is not exposed. Those skilled in the art can understand that, they can determine whether a specific amino acid site is modified by a PEG molecule through conventional technical means, for example, referring to the method listed in the "Detection of Polyethylene Glycol-Modified Sites" in Example 3 of the present disclosure. In short, the method includes: 1) digesting the non-pegylated and pegylated urate oxidases with one or more enzymes, for example, single-enzyme digestion by using Lys-C or Trypsin, or double-enzyme digestion by using Lys-C and Trypsin; 2) separating the digested fragments by high performance liquid chromatography to generate chromatograms of non-pegylated and pegylated urate oxidases, that is, peptide maps; and 3) comparing the peptide maps of the non-pegylated and pegylated urate oxidases to obtain the difference therebetween, and in combination with a predetermined internal standard peptides, determining a relative proportion of reduction or disappearance of peaks of the peptide fragment where specific amino acid sites are located in the pegylated urate oxidase, and further determining whether the specific amino acid sites on the peptide fragment are modified by PEG. Specifically, in Example 3 of the present disclosure, the relative proportion of the reduction or disappearance of the peak area of the peptide fragment where the specific amino acid sites are located can be calculated according to the following equation:

$$P\,(\%) = (A2- A1)/A2 \times 100\%,$$

where $A1 = A0 \times t$.

A0 is a measured peak area of a peptide fragment where a specific amino acid site of the modified protein to be tested is located, and t is an average ratio of a peak area of the internal reference peptide fragment in a PHC peptide map to that in the peptide map of the modified protein to be tested; P (%) represents a relative proportion of reduction or disappearance of the peak area of the peptide fragment where the specific amino acid site is located, A2 is a peak area of the peptide fragment where the specific amino acid site is located in the PHC peptide map, and A1 is an internal reference-converted peak area of the peptide fragment where the specific amino acid site is located in the peptide map of the modified protein to be tested.

It should be understood that, within the scope of the present disclosure, the above-mentioned technical features of the present disclosure and the various technical features specifically described as below (such as in the embodiments) can be combined with each other to form a new or preferred technical solution, which can be understood more clearly by referring to the following examples. Due to limited length, the described examples are for illustrative purposes only and are not intended to limit the present disclosure.

Hereinafter, the embodiments of the present disclosure will be described in further detail, and examples of the embodiments are illustrated in the accompanying drawings. The following embodiments described with reference to the accompanying drawings are illustrative, and are intended to explain the present disclosure, but should not be understood as limitations on the present disclosure.

**Example 1: Preparation of Recombinant Urate Oxidase**

1.1 Construction of genes and expression plasmids for uricase expression

According to the usage preference data of E. coli codon, in combination with factors such as codon preference and GC content, cDNA sequence of the uricase protein (referred to as PHC) (SEQ ID NO:1) was designed, and the whole gene was synthesized and named as pUC-57-PHC plasmid. Nde I and BamH I were used as the target gene insertion sites, and the pET-30a plasmid was used as the expression vector (pET-30a-PHC).

1.2 Transformation of expression plasmids into bacterial host cells

The expression vector pET-30a-PHC was introduced into E. coli BL21 (DE3) by CaCl2 method, high expression clones were screened through resistance screening with Kanamycin, and the original seed bank strain (E3B) was preserved. These steps were performed in accordance with the commonly used methods in the field of molecular biology.

1.3 Preparation of recombinant urate oxidase

The transformed and engineered strains were fermented and expressed in a fermentor, under the control conditions: first culturing at 30°C and pH 7.2 to an OD600=30 or higher, adding IPTG to 0.5 mmol/L, and continuing to induce for 3 hours or more to allow urate oxidase to accumulate. The cells were collected by centrifugation, and then preserved below -15°C.
The frozen bacteria were taken and suspended in a buffer of 25 mmol/L Tris and 5 mmol/L EDTA, at a suspension ratio of 1:10 (W/V). After breaking the bacterial cells with high pressure, the urate oxidase precipitate was collected by centrifugation, the precipitate was washed once with 50 mmol/L $NaHCO_3$, and the enriched uricase precipitate was suspended in a $Na_2HCO_3$ buffer (100 mmol/L, pH 9.7 to 10.3) at a suspension ratio of 1:50 (W/V), following by stirring overnight at room temperature to dissolve, and then centrifuging to collect the supernatant.
Urate oxidase was further purified through several chromatographic steps. The purity detected by SDS-PAGE was 95% or greater, and the purity detected by Superdex 200 column was greater than 95%, with no aggregate form. The protein concentration was determined by Lowry method, and the activity of the urate oxidase was measured by spectrophotometry, where 1 unit (U) of enzyme activity was defined as the amount of enzyme required to convert 1 $\mu$mol of uric acid per minute under the optimal reaction temperature of 37°C and the optimal buffer condition at pH 9.0.

**Example 2: Preparation of Pegylated Urate Oxidase**

N-succinimidyl propionate PEG having a molecular weight of 5KD (5K-PEG-SPA) was dissolved with 2 mmol/L HCl into 200 mmol/L PEG solution, which, after the dissolving, was added to a carbonate buffer solution containing the urate oxidase dissolved therein and having a carbonate concentration of 0.1 to 0.3mol/L, pH 10.0, according to a molar ratio ranging from 1:55 to 1:95 (urate oxidase: 5K-PEG-SPA), in which the molar ratio of urate oxidase to 5K-PEG-SPA was calculated based on the monomer form, that is, the molar ratio ranging from 1:55 to 1:95 refers to a molar ratio of urate oxidase in a monomer form to 5K-PEG-SPA, thereby allowing a coupling reaction between PEG and urate oxidase. The coupling reaction required stirring at 5 to 30°C for 60 minutes or more, until the PEG coupling degree no longer changed with time. After the reaction was finished, the PEG not participating in the modification and by-products were removed from the reaction by ultrafiltration and/or chromatography. A suitable molecular sieve chromatography medium was used to separate and remove modified by-products. Finally, the 5K modified pegylated urate oxidase (referred to as PU5) was obtained through sterile filtration.

**Example 3: Characteristic Analysis of Pegylated Urate Oxidase**

3.1 Detection of average modification degree and enzyme activity

The protein concentration was determined by using Lowry method, and the activity of the polyethylene glycol-modified urate oxidase was determined by using spectrophotometer. The maximum ultraviolet absorption wavelength of uric acid, i.e., the substrate of uricase, was 293nm, and the maximum ultraviolet absorption wavelength of the product allantoin was 224nm. Within a certain concentration range, the absorption value of uric acid at 293nm was in direct proportion to its concentration. The quantitative determination of uric acid can be carried out by spectrophotometer. The specific process was as follows: the UV-Vis spectrophotometer was turned on, the wavelength was adjusted to 293nm, and the water bath circulation system of the instrument was turned on to keep the temperature at 37°C. Sodium tetraborate buffer was used as a blank control, and zero point was calibrated; 2.95ml of substrate reaction solution (0.1 mol/L sodium

tetraborate, 100 $\mu$mol/L uric acid, pH 9.5, preheated to 37°C) was added in a quartz cuvette, then 50 $\mu$l of the test sample was added and mixed quickly to measure the absorption value at 293nm. The change of the absorbance at 293nm was continuously measured; a degradation concentration of uric acid was calculated according to C=A/$\varepsilon$L (where A is a absorbance of a specific concentration of uric acid at 293 nm, $\varepsilon$ is a molar extinction coefficient of uric acid, L is an optical path of the cuvette, and C is a molar concentration of uric acid). The enzyme activity was calculated. The enzyme activity was defined as that, at the optimum reaction temperature of 37°C and the optimum reaction pH of 9.5, the amount of enzyme required to convert 1 $\mu$mol of uric acid into allantoin per minute is defined as one activity unit (U).

SEC-HPLC connected in series with UV/RI (a combination of ultraviolet and refractive index detector) was used to detect the average modification degree of polyethylene glycol-modified urate oxidase. Based on the fact that protein has a maximum absorption peak at ultraviolet 280nm, PEG has no absorption at this wavelength, and the absorption value of the protein and the absorption value of PEG within a certain range by the differential refractive index detector are proportional to the respective concentrations, the content of PEG portion and the content of protein portion in the pegylated urate oxidase can be obtained using an external standard method with PEG reference substance and PHC physical and chemical reference substance, and thus the number of PEG molecules on each urate oxidase monomer, i.e., the average modification degree, can be obtained by the following calculation method.

Average modification degree of PEG-modified urate oxidase = (relative molecular weight of urate oxidase subunit × amount of PEG in sample)/(relative molecular weight of PEG × amount of protein in sample).

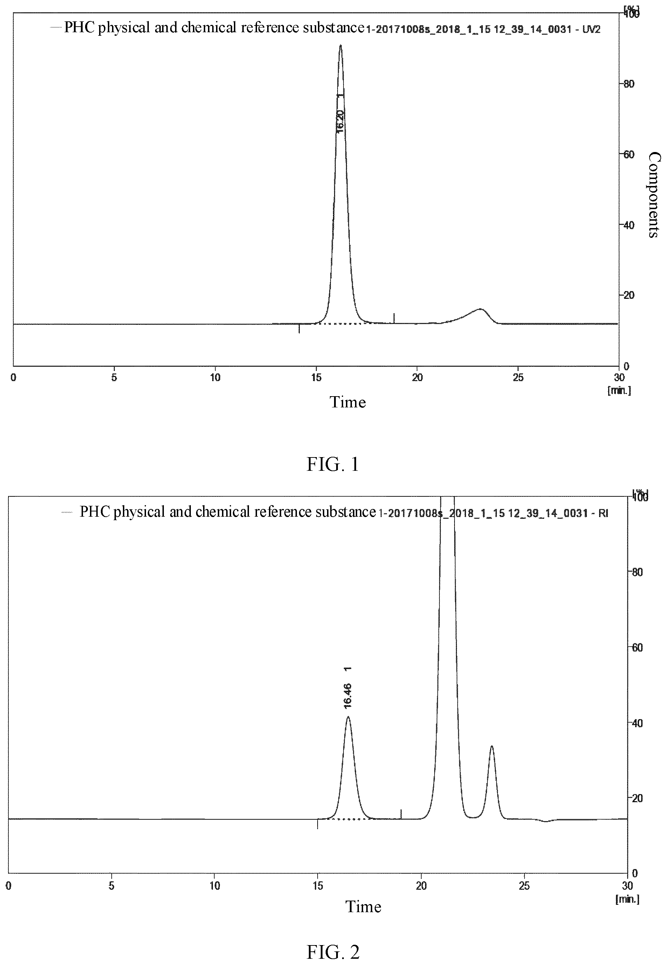

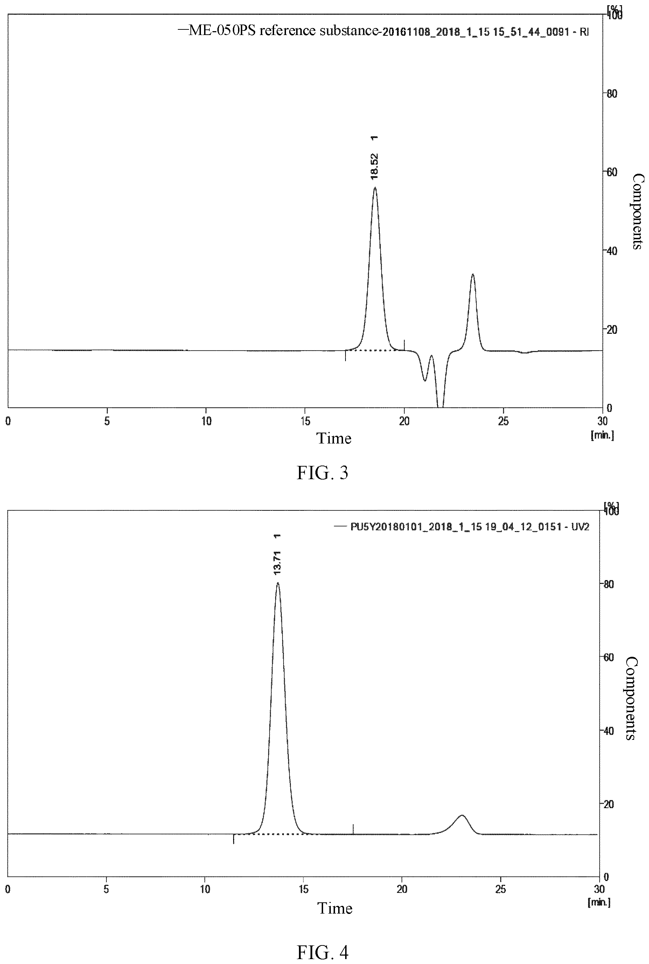

The SEC-HPLC-UV/RI detection spectrums of PHC physical and chemical reference substance, PEG reference substance, and PU5 modified product are illustrated in FIG. 1 to FIG. 5.

Under different feed ratios in Example 2, the enzyme activity and average modification degree of the obtained polyethylene glycol-modified urate oxidase are shown in Table 1.

[Table 1] Enzyme activity under different feed ratios

| Molar feed ratio of protein to 5K-PEG | Enzyme activity | Enzyme activity retention | Average modification degree |
|---|---|---|---|
| Unmodified protein | 11.4 U/mg | 100% | 0 |
| 1: 55 | 11.7 U/mg | 102.6% | 11.3 |
| 1: 68 | 12.1 U/mg | 106.1% | 11.7 |
| 1: 82 | 11.9 U/mg | 104.4% | 12.1 |
| 1: 95 | 12.3 U/mg | 107.9% | 12.2 |
| Note: the average modification degree represents the number of PEG molecules bound to each urate oxidase monomer | | | |

It can be seen from Table 1 that the average modification degree of the polyethylene glycol-modified urate oxidase of the present disclosure stablizes at 11 or more, and has an enzyme activity higher than that of unmodified urate oxidase, the enzyme activity retention is high, the enzyme activity is even increased instead of decreasing, and the enzyme activity is relatively stable, which is completely different from the marketed drug Krystexx (pegloticase). According to the content disclosed in the patent of Savient (CN1264575C, Figure 2A to Figure 3B) and the common knowledge of those skilled in the art, the enzyme activity decreases significantly with the increasing modification degree of 5kD PEG. However, unexpectedly, with the average modification degree of more than 11, the polyethylene glycol-modified uricase obtained in the present disclosure does not have its enzyme activity changed significantly compared with the unmodified state. Therefore, the polyethylene glycol-modified urate oxidase of the present disclosure has a higher average modification degree of polyethylene glycol compared with the marketed drugs, and also achieves unexpected technical effects in terms of enzyme activity retention, which may be attributed to the differences in PEG modification degree or modification sites of polyethylene glycol-modified urate oxidase, according to Applicant's speculation.

The similar technical effects were obtained when Applicant modified the proteins of SEQ ID NO: 2 to SEQ ID NO: 7. That is, when the 5kd modification degree is greater than 11, obtained is a pegylated urate oxidase having an enzyme activity that is not substantially reduced.

3.2 Detection of polyethylene glycol modification sites

In the following steps, Applicant performed modification site detection on the urate oxidase obtained in Example 2.

The PEG modification sites of polyethylene glycol-modified urate oxidase can detected by first performing enzyme digestion of the non-pegylated and pegylated urate oxidases with one or more enzymes and then performing chromatographic detection to obtain a chromatogram, i.e., peptide map for determination. The non-pegylated and pegylated urate oxidases can be digested through single-enzyme digestion (Lys-C or Trypsin) and/or double-enzyme digestion (Lys-C and Trypsin combined). The digested enzyme fragments were separated by reversed-phase column, and the modified sites of polyethylene glycol-modified urate oxidase were determined by the internal reference peptide fragment correction and comparison of the disappearance or reduction proportion of the peptide fragments.

Modification site analysis principle of trypsin and Lys-C double enzyme digestion quality peptide map: Lys-C can specifically digest the C-terminal of lysine (K); trypsin takes the basic amino acids, i.e., arginine (R) and lysine (K), as restriction enzyme cutting sites, and specifically digests the C-terminal peptide bond. Comparing the changes of the corresponding peptide fragments before and after digestion in PHC and PU5, and with reference to the internal standard peptide fragments, the relative proportion of the reduction or disappearance of the PEG-modified peptide fragments can be analyzed and determined. Through the relative proportion of the reduction or disappearance of the peptide fragment, it can be determined whether the lysine site on the peptide fragment is modified by PEG and the modified proportion. It should be pointed out that PEG modification is a non-uniform modification, and a high modification proportion of a site can be regarded as that this site is modified.

Details as follows:

(1) Sample processing: urate oxidase and pegylated urate oxidase were taken and respectively diluted to 1 mg/ml with enzyme digestion buffer (25mmol/L Tris-HCl, 20% acetonitrile, pH 9.0), and 100 $\mu$l of each dilute solution was taken, added with 2 $\mu$l of Lys-C, and digested at 37°C for 4 hours. Subsequently, the solution was transferred to a pancreatin reaction tube (in a ratio of 1:100), digestion was continued at 37°C for 2 hours, 4 $\mu$l of TCEP reducing solution was added, the reaction continued for 30 minutes, and then 10 $\mu$l of 1 mol/L hydrochloric acid solution was added to terminate the reaction.

(2) Analysis conditions:

Instrument: Thermo Ultimate 3000 HPLC and MSQ Plus;
Chromatographic column: Welch Materials $\mu$ltimate®XB-C18 (4.6 mm × 250 mm, 5 $\mu$m), Welch;
Analysis conditions: A solution (aqueous solution containing 0.1% TFA), solution B (acetonitrile solution containing 0.1% TFA);
Gradient: 0 to 70 min, B from 3 to 70%;
LC detection wavelength: 214 nm;
Ion source: ESI;
Ion type: positive ion;
Cone voltage: 50V;
Scanning range: 300 to 2000 Da;
Scan time: 1S;
Post column flow splittered to MS: about 0.3ml/min.
100$\mu$l of the sample was injected, and the chromatogram was recorded.

(3) Results processing:
The chromatograms (peptide maps) of urate oxidase and pegylated urate oxidase were compared, and the relative proportion of area reduction of the differential peptide fragments.

(4) The experimental results are shown in Table 2 to Table 5, and FIG. 6 to FIG. 7.

[Table 2] List of peptide fragments of PHC after digestion with Lys-C

| Peptide fragment | Restriction enzym e cutting site | Sequence | Theoretical molecular weight (Da) | Measured molecular weight |
|---|---|---|---|---|
| T1 | 3 | TYK | 410.47 | 410.2 |
| T2 | 4 | K | 146.189 | / |
| T3 | 17 | NDEVEFVRTGYGK | 1513.627 | / |

(continued)

| Peptide fragment | Restriction enzyme cutting site | Sequence | Theoretical molecular weight (Da) | Measured molecular weight |
|---|---|---|---|---|
| T2+T3 | | KNDEVEFVRTGYGK | 1641.089 | 1642.2 |
| T4 | 21 | DMIK | 505.63 | 505.4 |
| T5 | 30 | VLHIQRDGK | 1065.241 | 1065 |
| T6 | 35 | YHSIK | 646.744 | 646.5 |
| T7 | 48 | EVATTVQLTLSSK | 1376.57 | / |
| T8 | 49 | K | 146.189 | / |
| T9 | 66 | DYLHGDNSDVIPTDTIK | 1903.032 | 1903 |
| T10 | 74 | NTVNVLAK | 858.005 | 857.7 |
| T11 | 76 | FK | 293.366 | 293.1 |
| T12 | 79 | GIK | 316.401 | 316.2 |
| T13 | 97 | SIETFAVTICEHFLSSFK | 2059.364 | 2059 |
| T14 | 112 | HVIRAQVYVEEVPWK | 1853.154 | 1852.8 |
| T15 | 116 | RFEK | 578.669 | 578.4 |
| T16 | 120 | NGVK | 416.478 | 417.2 |
| T17 | 152 | HVHAFIYTPTGTHFCEVEQIRNGPPVIHSGIK | 3586.088 | 3586.2 |
| T18 | 155 | DLK | 374.437 | 374.1 |
| T19 | 158 | VLK | 358.481 | 358.2 |
| T20 | 169 | TTQSGFEGFIK | 1214.34 | 1213.8 |
| T21 | 179 | DQFTTLPEVK | 1177.32 | 1176.8 |
| T22 | 190 | DRCFATQVYCK | 1333.543 | 1333.2 |
| T23 | 215 | WRYHQGRDVDFEATWDTVRSIVLQK | 3106.45 | 3106.5 |
| T24 | 222 | FAGPYDK | 796.878 | 796.5 |
| T25 | 231 | GEYSPSVQK | 994.069 | 993.7 |
| T26 | 266 | TLYDIQVLTLGQVPEIEDMEISLPNIHYLNIDMSK | 4046.66 | 4046.1 |
| T27 | 272 | MGLINK | 674.856 | / |
| T28 | 285 | EEVLLPLDNPYGK | 1486.685 | 1486.6 |

(continued)

| Peptide fragment | Restriction enzym e cutting site | Sequence | Theoretical molecular weight (Da) | Measured molecular weight |
|---|---|---|---|---|
| T27+ T28 | | MGLINK EEVLLPLDNPYGK | 2143.54 | 2143.2 |
| T29 | 291 | ITGTVK | 617.743 | 617.4 |
| T30 | 293 | RK | 302.377 | / |
| T31 | 298 | LSSRL | 574.678 | 574.4 |

[Table 3] List of peptide fragments of PHC after double-enzyme digestion with Lys-C and trypsin

| Peptide fragme nt | Sequence position | Sequence | Theoretical relative molecular weight [Da] | Measured molecular weight |
|---|---|---|---|---|
| T1 | 1-3 | TYK | 410.470 | 410.3 |
| T2 | 4 | K | 146.189 | / |
| T3 | 5-12 | NDEVEFVR | 1007.068 | / |
| T2+3 | 4-12 | KNDEVEFVR | | 1135.4 |
| T4 | 13-17 | TGYGK | 524.574 | 524.5 |
| T5 | 18-21 | DMIK | 505.630 | 505.5 |
| T6 | 22-27 | VLHIQR | 764.926 | 764.8 |
| T7 | 28-30 | DGK | 318.330 | / |
| T8 | 31-35 | YHSIK | 646.744 | 646.7 |
| T9 | 36-48 | EVATTVQLTLSSK | 1376.570 | / |
| T10 | 49 | K | 146.189 | / |
| T11 | 50-66 | DYLHGDNSDVIPTDTIK | 1903.032 | 1903.4 |
| T12 | 67-74 | NTVNVLAK | 858.005 | 857.9 |
| T13 | 75-76 | FK | 293.366 | 293.1 |
| T14 | 77-79 | GIK | 316.401 | / |
| T15 | 80-97 | SIETFAVTICEHFLSSFK | 2059.364 | 2059.6 |
| T16 | 98-101 | HVIR | 523.636 | 523.6 |
| T17 | 102-112 | AQVYVEEVPWK | 1347.534 | 1347.4 |
| T18 | 113 | R | 174.203 | / |
| T19 | 114-116 | FEK | 422.481 | / |
| T18+19 | 113-116 | RFEK | 578.684 | 578.6 |
| T20 | 117-120 | NGVK | 416.478 | 417.1 |
| T21 | 121-141 | HVHAFIYTPTGTHFCEV EQIR | 2485.802 | 2486.8 |
| T22 | 142-152 | NGPPVIHSGIK | 1118.301 | 1118.8 |
| T21+22 | 121-152 | | 3586.103 | 3587.7 |

(continued)

| Peptide fragment | Sequence position | Sequence | Theoretical relative molecular weight [Da] | Measured molecular weight |
|---|---|---|---|---|
| T23 | 153-155 | DLK | 374.437 | / |
| T24 | 156-158 | VLK | 358.481 | 358.3 |
| T25 | 159-169 | TTQSGFEGFIK | 1214.340 | 1214.2 |
| T26 | 170-179 | DQFTTLPEVK | 1177.320 | 1177.2 |
| T27 | 181-181 | DR | 289.291 | / |
| T28 | 182-190 | CFATQVYCK | 1062.267 | / |
| T27+28 | 181-190 | | 1333.558 | 1333.6 |
| T29 | 191-192 | WR | 360.416 | 360.1 |
| T30 | 193-197 | YHQGR | 659.702 | 659.6 |
| T31 | 198-209 | DVDFEATWDTVR | 1453.528 | 1453.6 |
| T32 | 210-215 | SIVLQK | 686.850 | 686.8 |
| T33 | 216-222 | FAGPYDK | 796.878 | 796.8 |
| T34 | 223-231 | GEYSPSVQK | 994.069 | 994.1 |
| T35 | 262-266 | TLYDIQVLTLGQVPEIE DMEISLPNIHYLNIDMS | 4046.660 | 4047 |
| T36 | 267-272 | MGLINK | 674.856 | 674.7 |
| T37 | 273-285 | EEVLLPLDNPYGK | 1486.685 | 1486.7 |
| T36+37 | | | 2143.541 | 2143.6 |
| T38 | 286-291 | ITGTVK | 617.743 | 617.7 |
| T39 | 292 | R | 174.203 | / |
| T40 | 293 | K | 146.189 | / |
| T41 | 294-297 | LSSR | 461.519 | 461.5 |
| T42 | 298 | L | 131.175 | / |

The calculation method of the reduction percentage of peak area of PU5 peptide fragments is as follows:
The following formula can be used to calculate the corresponding peak areas of PU5 peptide fragments at the concentration of PU5 same as the concentration of PHC:

$$A1 = A0 \times t$$

where A1 is a peak area of PU5 peptide fragments converted with two internal reference peptides, A0 is a measured peak area of peptide fragments of a PU5 peptide map, and t is an average value of a peak area ratio of T30 internal reference peptide fragment in the PHC peptide map to T30 internal reference peptide fragment in the PU5 peptide map and a peak area ratio of T31 internal reference peptide fragment in the PHC peptide map to T31 internal reference peptide fragment in the PU5 peptide map, that is, t is 0.588.

[Table 4] Comparison of PHC and PU5 internal reference peptide fragments

| Peptide fragment No. | Sequence | PHC peptide map | | PU5 peptide map | | Peak area ratio of PHC to PU5 | |
|---|---|---|---|---|---|---|---|
| | | Retention time | Peak area | Retention time | Peak area | Value | Mean |
| T30 | YHQGR | 7.5 | 13.4 | 7.467 | 22.9 | 0.585 | 0.588 |
| T31 | DVDFEAT WDTVR | 28.31 | 35.5 | 28.28 | 60.1 | 0.591 | |

With the peak area of peptide fragment converted with the internal reference and the peak area of PHC peptide map, the relative percentage of the reduction of the peak area of a certain peptide in the PU5 peptide map can be calculated based on the following equation:

$$P(\%) = (A_2 - A_1)/A_2 \times 100\%$$

where A2 is a peak area of a certain peptide fragment in the PHC peptide map, and A1 is a peak area of this peptide fragment in PU5 converted with internal reference.

[Table 5] Summary results of peptide fragments with reduced peak area in the peptide map after PU5 was digested with double enzymes

| Peptide fragment position | Peptide fragment sequence | Relative proportion of reduced peak area of peptide fragment |
|---|---|---|
| 1-3 | TYK | 100.00% |
| 4-12 | KNDEVEFVR | 94.07% |
| 31-35 | YHSIK | 100.00% |
| 75-76 | FK | 82.27% |
| 80-97 | SIETFAVTICEHFLSSFK | 100.00% |
| 102-112 | AQVYVEEVPWK | 100.00% |
| 113-116 | RFEK | 100.00% |
| 117-120 | NGVK | 100.00% |
| 121-152 | HVHAFIYTPTGTHFCEVEQIRN GPPVIHSGIK | 100.00% |
| 193-197 | YHQGR | Internal reference peptide fragment |
| 198-209 | DVDFEATWDTVR | Internal reference peptide fragment |
| 216-222 | FAGPYDK | 91.37% |
| 223-231 | GEYSPSVQK | 86.40% |
| 232-266 | TLYDIQVLTLGQVPEIEDMEISL PNIHYLNIDMSK | 100.00% |
| 273-285 | EEVLLPLDNPYGK | 100.00% |

Based on the analysis of the protein sequence (SEQ ID NO: 1) of the present example, the potential sites for urate oxidase modification include 31 sites, including T[1], K[3], K[4], K[17], K[21], K[30], K[35], K[48], K[49], K[66], K[74], K[76], K[79], K[97],

$K^{112}$, $K^{116}$, $K^{120}$, $K^{152}$, $K^{155}$, $K^{158}$, $K^{169}$, $K^{179}$, $K^{190}$, $K^{215}$, $K^{222}$, $K^{231}$, $K^{266}$, $K^{272}$, $K^{285}$, $K^{291}$, and $K^{293}$.

Based on the analysis of the modification sites of polyethylene glycol-modified urate oxidase obtained in Example 2, as shown in Table 2, Table 3, Table 4, Table 5 and FIG. 6, the sites with 90% or more disappearance of the peptide fragments after PU5 digestion include $K^3$, $K^4$, $K^{35}$, $K^{97}$, $K^{112}$, $K^{116}$, $K^{120}$, $K^{152}$, $K^{222}$, $K^{266}$, and $K^{285}$, the sites with 80% to 90% disappearance of the peptide fragments after PU5 digestion include $K^{76}$ and $K^{231}$. In PU5, these sites are all modified.

Moreover, Applicant found that the polyethylene glycol-modified urate oxidase of the present disclosure has more modification sites than the marketed drugs, and has significant differences. For example, through single enzyme digestion of the , it was found that, the disappearance ratio of the peptide fragments where the four sites, $K^{30}$, $K^{35}$, $K^{222}$, and $K^{231}$ are located in the polyethylene glycol-modified urate oxidase of the present disclosure is more than 80%. However, by analyzing the marketed analogous drug Krystexx (pegloticase) with the method, the peptide fragments where these four sites are located hardly disappeared, that is, the modification rate of the marketed analogous drug at the four sites $K^{30}$, $K^{35}$, $K^{222}$, and $K^{231}$ is much lower than that of the polyethylene glycol-modified urate oxidase of the present disclosure. In addition, the polyethylene glycol-modified urate oxidase of the present disclosure has significantly lower immunogenicity than the marketed drug, which may be related to the differences in the number of modification sites and the modification sites according to Applicant's speculation. Due to the differences in the modification sites and modification degree, the protection to the immunogenic site of the enzyme and the exposure of the active center of the enzyme in the body are both different. The above-mentioned differences may cause the difference in the biological properties of different modified enzymes in the body.

The in vivo drug evaluation of the polyethylene glycol-modified urate oxidase (PU5) of the present disclosure in animals will be described in detail as below. The pegloticase used in the experiment refers to the analogous drug that has been marketed with a batch number 5085B.

**Example 4: Study on in vivo pharmacodynamics of polyethylene glycol-modified urate oxidase**

4.1. In vivo efficacy evaluation of polyethylene glycol-modified urate oxidase in model rats

A chronic hyperuricemia rat model was induced by potassium oxazinate drinking water in combination with high uric acid feed, to evaluate the therapeutic effect of polyethylene glycol-modified urate oxidase (PU5) on chronic hyperuricemia in rats.

40 model rats were selected and randomly divided into 4 groups, i.e., a model group, a low-dose pegylated uricase administration group (0.3 mg/kg), a medium-dose pegylated uricase administration group (1.0 mg/kg), and a high-dose pegylated uricase administration group (3.0 mg/kg), 10 rats in each group; and additionally, 10 normal SD rats were selected as the blank control group. The model-establishing experiment was conducted for 5 consecutive weeks, and intramuscular administration was started 1 week after the start of the model establishing. The administration was performed and continued once a week for 4 consecutive weeks. The levels of serum uric acid, serum urea nitrogen, and serum creatinine of rats before the administration and 7 days after each administration were detected, and the histological changes of rat kidneys were observed after the experiment.

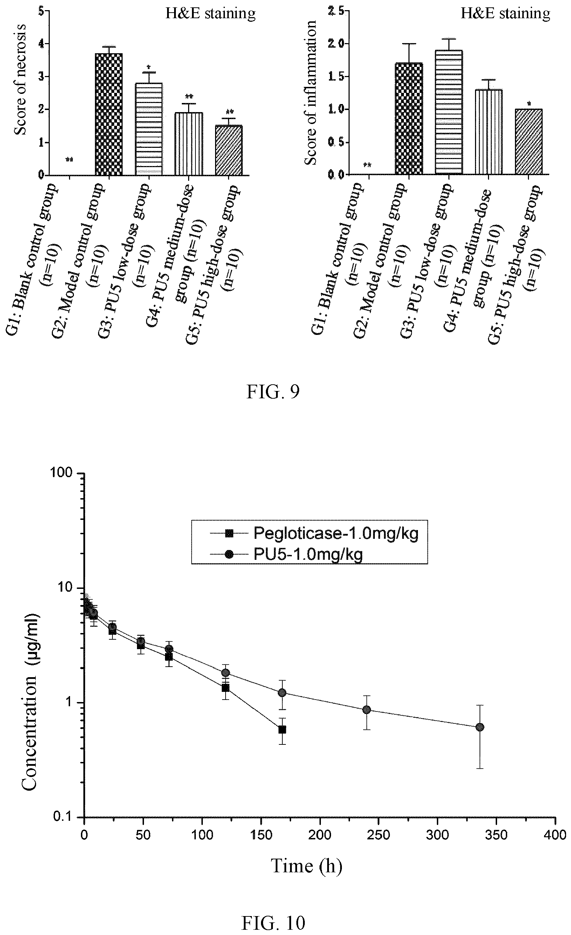

The results in FIG. 8 indicate that on days 7, 14, 21, 28, and 35 after the start of the model establishing, compared with the blank control group, the serum uric acid level of the model control group increased significantly, and the serum urea nitrogen, creatinine and uric acid of the rats in the model group were 2.73 times, 2.40 times and 7.83 times these in the blank group, respectively. From the perspective of kidney pathology (as shown in FIG. 9), the scores of renal tubule dilatation, necrosis, inflammation, and fibrosis of the model control group increased significantly, and the quantity of urate crystals also increased significantly. Both the medium and high doses of the test substance, pegylated uricase, significantly reduced the serum uric acid level, which exhibited a dose-dependent relationship. During the period from day 14 to day 35, the average serum uric acid level of the medium-dose group was maintained at 303.80 to 660.60 $\mu$mol/L, and the average serum uric acid level of the high-dose group was maintained at 153.70 to 403.40 $\mu$mol/L. Compared with the model group, the serum uric acid in the medium-dose group was reduced by 34.46% to 67.94%; the serum uric acid in the high-dose group was reduced by 65.67% to 83.78%. Compared with the model control group, each pegylated uricase administration group had significant ameliorating effects on the renal tubule dilatation, renal necrosis and inflammation.

4.2 Evaluation of single administration of polyethylene glycol-modified urate oxidase in rats

36 SD rats (half females and half males) were randomly divided into 6 groups (see Table 6), namely, a marketed drug Pegloticase intravenous injection group, a Pegloticase intramuscular injection group, a polyethylene glycol-modified urate oxidase intravenous injection group, as well as low-dose, medium-dose and high-dose polyethylene glycol-modified urate oxidase intramuscular injection groups (0.5 mg/kg, 1.0 mg/kg, 2.0 mg/kg). The specific administration regimens and doses are shown in Table 6. PK and PD were detected by taking the blood from the jugular vein.

[Table 6] Animal grouping and dose design

| No. | Group | Administration route | Administration frequency | Administration dose (mg/kg) | Administration concentration (mg/ml) | Administration volume (ml/kg) | Quantity of animals | |
|---|---|---|---|---|---|---|---|---|
| | | | | | | | Male | Female |
| 1 | Pegloticase intravenous injection group | Intravenous injection | One time | 1.0 | 0.1 | 10.0 | 3 | 3 |
| 2 | Pegloticase intramuscular injection group | Intramuscular injection | One time | 1.0 | 1.0 | 1.0 | 3 | 3 |
| 3 | PU5 intravenous injection group | Intravenous injection | One time | 1.0 | 0.1 | 10.0 | 3 | 3 |
| 4 | PU5 low-dose intramuscular injection group | Intramuscular injection | One time | 0.5 | 0.5 | 1.0 | 3 | 3 |
| 5 | PU5 medium-dose intramuscular injection group | Intramuscular injection | One time | 1.0 | 1.0 | 1.0 | 3 | 3 |
| 6 | PU5 high-dose intramuscular injection group | Intramuscular injection | One time | 2.0 | 2.0 | 1.0 | 3 | 3 |

4.2.1. Pharmacokinetic comparison

All the SD rats, before the administration, each had a serum drug concentration level lower than the lower limit of quantification (LLOQ: 312.500 ng/mL), and after one single intramuscular injection of 0.5 mg/kg, 1.0 mg/kg, and 2.0 mg/kg of the drug , in a period from 0h to 168h (0 to 7 days), the serum drug concentration of the pegylated uricase injection (PU5) was dose-dependent and had an overall level increasing with the increase in the administered dose; and 168 hours later, the blood drug concentration of the pegloticase intramuscular administration group was lower than the lower limit of quantification, and the blood drug concentration of the PU5 intramuscular administration group maintained for more than 240h.

After administration, for each group of 1.0 mg/kg Pegloticase intravenous injection and intramuscular injection groups, 1.0 mg/kg pegylated uricase intravenous injection group, as well as 0.5 mg/kg, 1.0 mg/kg, and 2.0 mg/kg pegylated uricase intramuscular injection groups, , a ratio of Cmax (C5min) of female SD rats to male SD rats was in the range of 0.75 to 0.99, a ratio of AUClast of female SD rats to male SD rats was in the range of 0.54 to 0.94, and a ratio of AUC0-∞ of female SD rats to male SD rats was in the range of 0.58 to 0.97. It can be seen that there is no significant gender difference in the exposure levels of Pegloticase and the pegylated uricase (PU5) injections in SD rats.

However, when the SD rats were administered with the same dose (1.0 mg/kg), AUClast of the marketed drug Pegloticase intravenous administration group was 426.48 ± 65.34, AUClast of the Pegloticase intramuscular injection group was 264.19 ± 78.22; and AUClast of the PU5 injection intravenous administration group was 565.61 ± 161.60, the AUClast of the PU5 intramuscular injection group was 337.86 ± 227.34. Under the same dose and administration mode, the AUClast of PU5 was higher than that of the marketed drug Pegloticase.

When the SD rats were administered with the same dose (1.0 mg/kg), t1/2(h) of the marketed drug Pegloticase intravenous administration group was 49.51 ± 8.12, t1/2(h) of the Pegloticase intramuscular administration group was 55.21 ± 13.50, t1/2(h) of the PU5 injection intravenous administration group was 86.12 ± 33.82, and the t1/2(h) of the PU5 intramuscular administration group was 60.45 ± 21.37. Under the same dose and administration mode, the t1/2(h) of PU5 was longer than that of the marketed drug Pegloticase.

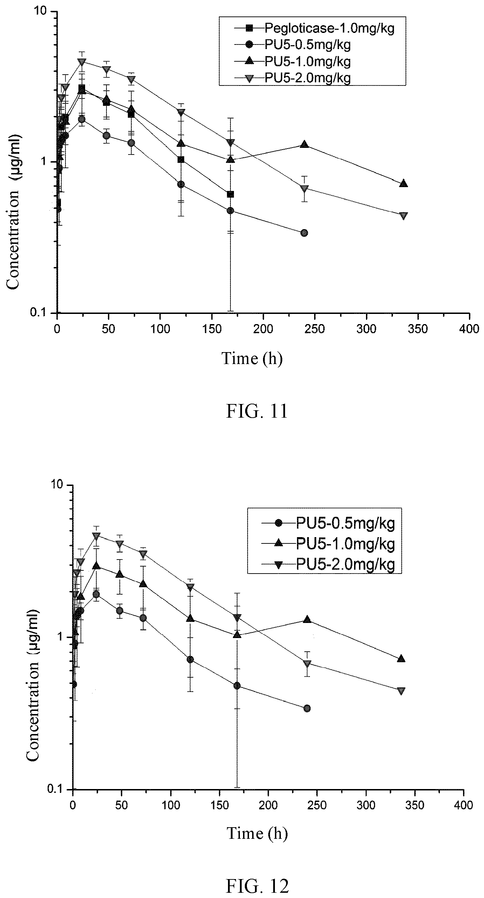

The above pharmacokinetic results are shown in Tables 7 to 12, and FIG. 10 to FIG. 12.

[Table 7] Individual blood drug concentration data and statistical analysis data when the SD rats received a single intravenous injection of 1.0 mg/kg Pegloticase (unit: $\mu$g/mL)

| Sampling time (h) | Male | | | | | | Female | | | | | | Female+Male | | |
|---|---|---|---|---|---|---|---|---|---|---|---|---|---|---|---|
| | 1M001 | 1M002 | 1M003 | N | Mean | SD | 1F001 | 1F002 | 1F003 | N | Mean | SD | N | Mean | SD |
| 0 | BLQ | BLQ | BLQ | 0 | / | / | BLQ | BLQ | BLQ | 0 | / | / | 0 | / | / |
| 0.08333 | 8.03 | 7.466 | 8.078 | 3 | 7.858 | 0.340 | 6.495 | 6.402 | 7.828 | 3 | 6.908 | 0.798 | 6 | 7.383 | 0.756 |
| 0.5 | 8.042 | 7.352 | 7.926 | 3 | 7.773 | 0.369 | 6.257 | 6.141 | 7.618 | 3 | 6.672 | 0.821 | 6 | 7.223 | 0.830 |
| 2 | 5.917 | 7.235 | 6.914 | 3 | 6.689 | 0.687 | 6.056 | 5.875 | 6.836 | 3 | 6.256 | 0.511 | 6 | 6.472 | 0.591 |
| 4 | 7.598 | 7.047 | 6.757 | 3 | 7.134 | 0.427 | 5.595 | 4.922 | 7.164 | 3 | 5.894 | 1.150 | 6 | 6.514 | 1.031 |
| 8 | 7.144 | 5.852 | 6.492 | 3 | 6.496 | 0.646 | 5.005 | 4.121 | 5.748 | 3 | 4.958 | 0.815 | 6 | 5.727 | 1.069 |
| 24 | 4.992 | 3.923 | 4.469 | 3 | 4.461 | 0.535 | 3.764 | 3.341 | 4.862 | 3 | 3.989 | 0.785 | 6 | 4.225 | 0.654 |
| 48 | 3.552 | 2.934 | 3.304 | 3 | 3.263 | 0.311 | 2.988 | 2.415 | 3.836 | 3 | 3.080 | 0.715 | 6 | 3.172 | 0.503 |
| 72 | 3.009 | 2.271 | 2.422 | 3 | 2.567 | 0.390 | 2.223 | 1.994 | 3.103 | 3 | 2.440 | 0.585 | 6 | 2.504 | 0.450 |
| 120 | 1.522 | 1.483 | 1.246 | 3 | 1.417 | 0.149 | 0.985 | 1.098 | 1.734 | 3 | 1.272 | 0.404 | 6 | 1.345 | 0.284 |
| 168 | 0.652 | 0.629 | 0.316 | 3 | 0.532 | 0.188 | 0.497 | 0.672 | 0.726 | 3 | 0.632 | 0.120 | 6 | 0.582 | 0.151 |
| 240 | BLQ | BLQ | BLQ | 0 | / | / | BLQ | BLQ | BLQ | 0 | / | / | 0 | / | / |
| 336 | BLQ | BLQ | BLQ | 0 | / | / | BLQ | BLQ | BLQ | 0 | / | / | 0 | / | / |
| Note: "/" means no relevant information. | | | | | | | | | | | | | | | |

[Table 8] Individual blood drug concentration data and statistical analysis data when the SD rats received a single intravenous injection of 1.0 mg/kg pegylated uricase (unit: μg/mL)

| Sampling time | Male | | | | | | Female | | | | | | Female+Male | | |
|---|---|---|---|---|---|---|---|---|---|---|---|---|---|---|---|
| (h) | 3M001 | 3M002 | 3M003 | N | Mean | SD | 3F001 | 3F002 | 3F003 | N | Mean | SD | N | Mean | SD |
| 0 | BLQ | BLQ | BLQ | 0 | / | / | BLQ | BLQ | BLQ | 0 | / | / | 0 | / | / |
| 0.08333 | 7.364 | 9.941 | 7.74 | 3 | 8.348 | 1.392 | 7.236 | 5.991 | 6.657 | 3 | 6.628 | 0.623 | 6 | 7.488 | 1.348 |
| 0.5 | 7.316 | 9.469 | 7.693 | 3 | 8.159 | 1.150 | 7.051 | 5.513 | 6.36 | 3 | 6.308 | 0.770 | 6 | 7.234 | 1.340 |
| 2 | 7.742 | 9.084 | 7.338 | 3 | 8.055 | 0.914 | 6.063 | 5.522 | 6.44 | 3 | 6.008 | 0.461 | 6 | 7.032 | 1.294 |
| 4 | 7 | 8.837 | 6.997 | 3 | 7.611 | 1.061 | 6.508 | 5.735 | 6.288 | 3 | 6.177 | 0.398 | 6 | 6.894 | 1.064 |
| 8 | 6.628 | 7.43 | 6.61 | 3 | 6.889 | 0.468 | 5.387 | 4.85 | 5.52 | 3 | 5.252 | 0.355 | 6 | 6.071 | 0.971 |
| 24 | 4.672 | 5.628 | 4.746 | 3 | 5.015 | 0.532 | 4.291 | 3.919 | 4.129 | 3 | 4.113 | 0.187 | 6 | 4.564 | 0.609 |
| 48 | 3.307 | 4.264 | 3.497 | 3 | 3.689 | 0.507 | 3.406 | 3.042 | 3.014 | 3 | 3.154 | 0.219 | 6 | 3.422 | 0.456 |
| 72 | 2.933 | 3.762 | 3.124 | 3 | 3.273 | 0.434 | 2.859 | 2.596 | 2.319 | 3 | 2.591 | 0.270 | 6 | 2.932 | 0.494 |
| 120 | 1.986 | 2.279 | 1.989 | 3 | 2.085 | 0.168 | 1.604 | 1.617 | 1.454 | 3 | 1.558 | 0.091 | 6 | 1.822 | 0.313 |
| 168 | 1.268 | 1.742 | 1.391 | 3 | 1.467 | 0.246 | 1.187 | 1.031 | 0.699 | 3 | 0.972 | 0.249 | 6 | 1.220 | 0.350 |
| 240 | 0.67 | 1.19 | 0.734 | 3 | 0.865 | 0.284 | BLQ | BLQ | BLQ | 0 | / | / | 3 | 0.865 | 0.284 |
| 336 | BLQ | 0.853 | 0.368 | 2 | 0.611 | 0.343 | BLQ | BLQ | BLQ | 0 | / | / | 2 | 0.611 | 0.343 |
| Note: "/" means no relevant information. | | | | | | | | | | | | | | | |

EP 3 967 754 A1

[Table 9] Individual blood drug concentration data and statistical analysis data when the SD rats received a single intramuscular injection of 1.0 mg/kg Pegloticase (unit: $\mu$g/mL)

| Sampling time | Male | | | | | | Female | | | | | | Female+Male | | |
|---|---|---|---|---|---|---|---|---|---|---|---|---|---|---|---|
| (h) | 2M001 | 2M002 | 2M003 | N | Mean | SD | 2F001 | 2F002 | 2F003 | N | Mean | SD | N | Mean | SD |
| 0 | BLQ | BLQ | BLQ | 0 | / | / | BLQ | BLQ | BLQ | 0 | / | / | 0 | / | / |
| 0.5 | 0.652 | BLQ | 0.581 | 2 | 0.617 | 0.050 | 0.388 | BLQ | BLQ | 1 | 0.388 | / | 3 | 0.540 | 0.137 |
| 2 | 1.337 | 1.249 | 1.62 | 3 | 1.402 | 0.194 | 1.172 | 1.135 | BLQ | 2 | 1.154 | 0.026 | 5 | 1.303 | 0.194 |
| 4 | 2.298 | 1.699 | 2.348 | 3 | 2.115 | 0.361 | 1.812 | 1.371 | 0.773 | 3 | 1.319 | 0.521 | 6 | 1.717 | 0.593 |
| 8 | 2.56 | 2.058 | 2.396 | 3 | 2.338 | 0.256 | 1.915 | 1.657 | 1.273 | 3 | 1.615 | 0.323 | 6 | 1.977 | 0.474 |
| 24 | 3.808 | 3.235 | 3.309 | 3 | 3.451 | 0.312 | 2.947 | 2.808 | 2.493 | 3 | 2.749 | 0.233 | 6 | 3.100 | 0.456 |
| 48 | 3.188 | 2.618 | 2.749 | 3 | 2.852 | 0.299 | 2.317 | 2.279 | 1.729 | 3 | 2.108 | 0.329 | 6 | 2.480 | 0.495 |
| 72 | 2.694 | 2.263 | 2.211 | 3 | 2.389 | 0.265 | 1.984 | 2.016 | 1.261 | 3 | 1.754 | 0.427 | 6 | 2.072 | 0.471 |
| 120 | 1.56 | 1.169 | 1.332 | 3 | 1.354 | 0.196 | 0.884 | 1.111 | 0.174 | 3 | 0.723 | 0.489 | 6 | 1.038 | 0.480 |
| 168 | BLQ | 0.341 | 0.869 | 2 | 0.605 | 0.373 | BLQ | 0.635 | BLQ | 1 | 0.635 | / | 3 | 0.615 | 0.265 |
| 240 | BLQ | BLQ | BLQ | 0 | / | / | BLQ | BLQ | BLQ | 0 | / | / | 0 | / | / |
| 336 | BLQ | BLQ | BLQ | 0 | / | / | BLQ | BLQ | BLQ | 0 | / | / | 0 | / | / |
| Note: "/" means no relevant information. | | | | | | | | | | | | | | | |

[Table 10] Individual blood drug concentration data and statistical analysis data when the SD rats received a single intramuscular injection of 1.0 mg/kg pegylated uricase
(unit: μg/mL)

| Sampling time | Male | | | | | | Female | | | | | | Female+Male | | |
|---|---|---|---|---|---|---|---|---|---|---|---|---|---|---|---|
| (h) | 5M001 | 5M002 | 5M003 | N | Mean | SD | 5F001 | 5F002 | 5F003 | N | Mean | SD | N | Mean | SD |
| 0 | BLQ | BLQ | BLQ | 0 | / | / | BLQ | BLQ | BLQ | 0 | / | / | 0 | / | / |
| 0.5 | BLQ | 1.421 | 0.328 | 2 | 0.875 | 0.773 | BLQ | BLQ | BLQ | 0 | / | / | 2 | 0.875 | 0.773 |
| 2 | BLQ | 2.295 | 0.923 | 2 | 1.609 | 0.970 | 0.593 | 0.905 | 0.674 | 3 | 0.724 | 0.162 | 5 | 1.078 | 0.695 |
| 4 | 0.729 | 2.897 | 1.648 | 3 | 1.758 | 1.088 | 1.356 | 1.222 | 0.762 | 3 | 1.113 | 0.312 | 6 | 1.436 | 0.798 |
| 8 | 1.305 | 3.628 | 2.054 | 3 | 2.329 | 1.186 | 1.559 | 1.249 | 1.266 | 3 | 1.358 | 0.174 | 6 | 1.844 | 0.926 |
| 24 | 2.408 | 4.617 | 3.069 | 3 | 3.365 | 1.134 | 3.01 | 2.339 | 2.216 | 3 | 2.522 | 0.427 | 6 | 2.943 | 0.895 |
| 48 | 2.068 | 3.877 | 2.4 | 3 | 2.782 | 0.963 | 2.739 | 2.298 | 2.189 | 3 | 2.409 | 0.291 | 6 | 2.595 | 0.668 |
| 72 | 1.76 | 3.606 | 2.027 | 3 | 2.464 | 0.998 | 2.385 | 1.761 | 1.863 | 3 | 2.003 | 0.335 | 6 | 2.234 | 0.712 |
| 120 | 1.042 | 2.9 | 1.107 | 3 | 1.683 | 1.054 | 1.169 | 0.811 | 0.926 | 3 | 0.969 | 0.183 | 6 | 1.326 | 0.782 |
| 168 | 0.479 | 2.419 | 0.631 | 3 | 1.176 | 1.079 | 0.595 | BLQ | BLQ | 1 | 0.595 | / | 4 | 1.031 | 0.928 |
| 240 | BLQ | 1.303 | BLQ | 1 | 1.303 | / | BLQ | BLQ | BLQ | 0 | / | / | 1 | 1.303 | / |
| 336 | BLQ | 0.719 | BLQ | 1 | 0.719 | / | BLQ | BLQ | BLQ | 0 | / | / | 1 | 0.719 | / |
| Note: "/" means no relevant information. | | | | | | | | | | | | | | | |

[Table 11] Average pharmacokinetic parameters of SD rats after a single intravenous injection of Pegloticase and pegylated uricase injections

| Dose | Gender | Parameter | $t_{1/2}$ | $C_{5min}$ | $AUC_{last}$ | $AUC_{0-\infty}$ | Vz | Cl | $MRT_{last}$ |
|---|---|---|---|---|---|---|---|---|---|
| (mg/kg) | | | (h) | ($\mu$g/mL) | (h*$\mu$g/mL) | (h*$\mu$g/mL) | (mL/kg) | (mL/h/kg) | (h) |
| 1.0 (Pegloticase) | Male | N | 3 | 3 | 3 | 3 | 3 | 3 | 3 |
| | | Mean | 45.70 | 7.86 | 448.57 | 484.58 | 136.90 | 2.08 | 52.36 |
| | | SD | 7.57 | 0.34 | 42.16 | 46.96 | 26.57 | 0.19 | 2.58 |
| | Female | N | 3 | 3 | 3 | 3 | 3 | 3 | 3 |
| | | Mean | 53.32 | 6.91 | 404.38 | 453.63 | 173.91 | 2.26 | 54.64 |
| | | SD | 7.98 | 0.80 | 86.21 | 90.21 | 43.64 | 0.40 | 2.62 |
| | Female+Male | N | 6 | 6 | 6 | 6 | 6 | 6 | 6 |
| | | Mean | 49.51 | 7.39 | 426.48 | 469.11 | 155.40 | 2.17 | 53.50 |
| | | SD | 8.12 | 0.76 | 65.34 | 66.52 | 38.14 | 0.30 | 2.64 |
| 1.0 (PU5) | Male | N | 3 | 3 | 3 | 3 | 3 | 3 | 3 |
| | | Mean | 105.16 | 8.35 | 692.29 | 794.77 | 186.76 | 1.31 | 90.87 |
| | | SD | 41.08 | 1.39 | 128.22 | 197.50 | 24.94 | 0.29 | 14.06 |
| | Female | N | 3 | 3 | 3 | 3 | 3 | 3 | 3 |
| | | Mean | 67.09 | 6.63 | 438.93 | 535.17 | 180.63 | 1.88 | 58.58 |
| | | SD | 9.24 | 0.62 | 26.51 | 59.13 | 11.64 | 0.21 | 2.76 |
| | Female+Male | N | 6 | 6 | 6 | 6 | 6 | 6 | 6 |
| | | Mean | 86.12 | 7.49 | 565.61 | 664.97 | 183.70 | 1.59 | 74.73 |
| | | SD | 33.82 | 1.35 | 161.60 | 192.92 | 17.73 | 0.39 | 19.87 |

[Table 12] Average pharmacokinetic parameters of SD rats after a single intramuscular injection of Peglocticase and pegylated uricase injections

| Dose | Gender | Parameter | $t_{1/2}$ | $T_{max}$ | $C_{max}$ | $AUC_{last}$ | $AUC_{0-\infty}$ | $Vz\_F$ | $Cl\_F$ | $MRT_{last}$ |
|---|---|---|---|---|---|---|---|---|---|---|
| (mg/kg) | | | (h) | (h) | ($\mu$g/mL) | (h*$\mu$g/mL) | (h*$\mu$g/mL) | (mL/kg) | (mL/h/kg) | (h) |
| 1.0 (Pegloticase) | Male | N | 3 | 3 | 3 | 3 | 3 | 3 | 3 | 3 |
| | | Mean | 58.31 | 24.00 | 3.45 | 318.23 | 405.13 | 203.75 | 2.54 | 60.57 |
| | | SD | 20.10 | 0.00 | 0.31 | 15.37 | 80.13 | 42.90 | 0.54 | 6.54 |
| | Female | N | 3 | 3 | 3 | 3 | 3 | 3 | 3 | 3 |
| | | Mean | 52.12 | 24.00 | 2.75 | 210.14 | 278.56 | 276.83 | 3.72 | 51.27 |
| | | SD | 4.78 | 0.00 | 0.23 | 79.35 | 60.90 | 50.57 | 0.91 | 15.28 |
| | Female+Male | N | 6 | 6 | 6 | 6 | 6 | 6 | 6 | 6 |
| | | Mean | 55.21 | 24.00 | 3.10 | 264.19 | 341.85 | 240.29 | 3.13 | 55.92 |
| | | SD | 13.50 | 0.00 | 0.46 | 78.22 | 94.12 | 57.97 | 0.93 | 11.68 |
| 0.5 (PU5) | Male | N | 3 | 3 | 3 | 3 | 3 | 3 | 3 | 3 |
| | | Mean | 63.57 | 24.00 | 1.93 | 181.10 | 233.11 | 199.06 | 2.21 | 60.26 |
| | | SD | 18.68 | 0.00 | 0.26 | 79.19 | 48.71 | 56.50 | 0.45 | 23.89 |
| | Female | N | 3 | 3 | 3 | 3 | 3 | 3 | 3 | 3 |
| | | Mean | 48.20 | 24.00 | 1.91 | 170.63 | 205.87 | 167.09 | 2.56 | 55.67 |
| | | SD | 17.38 | 0.00 | 0.14 | 41.99 | 61.41 | 21.47 | 0.67 | 11.48 |
| | Female+Male | N | 6 | 6 | 6 | 6 | 6 | 6 | 6 | 6 |
| | | Mean | 55.88 | 24.00 | 1.92 | 175.87 | 219.49 | 183.07 | 2.38 | 57.97 |
| | | SD | 18.20 | 0.00 | 0.19 | 56.98 | 51.77 | 42.05 | 0.54 | 16.95 |

(continued)

| Dose (mg/kg) | Gender | Parameter | $t_{1/2}$ (h) | $T_{max}$ (h) | $C_{max}$ ($\mu$g/mL) | $AUC_{last}$ (h*$\mu$g/mL) | $AUC_{0-\infty}$ (h*$\mu$g/mL) | Vz_F (mL/kg) | Cl_F (mL/h/kg) | $MRT_{last}$ (h) |
|---|---|---|---|---|---|---|---|---|---|---|
| 1.0 (PU5) | Male | N | 3 | 3 | 3 | 3 | 3 | 3 | 3 | 3 |
| | | Mean | 70.47 | 24.00 | 3.36 | 439.83 | 504.61 | 225.86 | 2.57 | 84.20 |
| | | SD | 28.55 | 0.00 | 1.13 | 307.66 | 344.91 | 54.21 | 1.32 | 31.10 |
| | Female | N | 3 | 3 | 3 | 3 | 3 | 3 | 3 | 3 |
| | | Mean | 50.44 | 24.00 | 2.52 | 235.90 | 293.04 | 252.46 | 3.46 | 58.28 |
| | | SD | 5.05 | 0.00 | 0.43 | 58.01 | 45.24 | 46.82 | 0.50 | 6.96 |
| | Female+Male | N | 6 | 6 | 6 | 6 | 6 | 6 | 6 | 6 |
| | | Mean | 60.45 | 24.00 | 2.94 | 337.86 | 398.83 | 239.16 | 3.02 | 71.24 |
| | | SD | 21.37 | 0.00 | 0.89 | 227.34 | 248.66 | 47.59 | 1.02 | 24.65 |
| 2.0 (PU5) | Male | N | 3 | 3 | 3 | 3 | 3 | 3 | 3 | 3 |
| | | Mean | 66.65 | 24.00 | 4.84 | 590.58 | 649.31 | 292.61 | 3.10 | 85.42 |
| | | SD | 20.11 | 0.00 | 0.46 | 59.68 | 55.26 | 64.39 | 0.27 | 19.91 |
| | Female | N | 3 | 3 | 3 | 3 | 3 | 3 | 3 | 3 |
| | | Mean | 72.51 | 32.00 | 4.55 | 537.05 | 628.72 | 339.98 | 3.22 | 79.26 |
| | | SD | 15.56 | 13.86 | 0.91 | 124.85 | 78.17 | 100.19 | 0.42 | 9.60 |
| | Female+Male | N | 6 | 6 | 6 | 6 | 6 | 6 | 6 | 6 |
| | | Mean | 69.58 | 28.00 | 4.70 | 563.81 | 639.01 | 316.30 | 3.16 | 82.34 |
| | | SD | 16.40 | 9.80 | 0.66 | 92.30 | 61.59 | 79.67 | 0.32 | 14.38 |

4.2.2. Comparison of in vivo efficacy (uric acid)

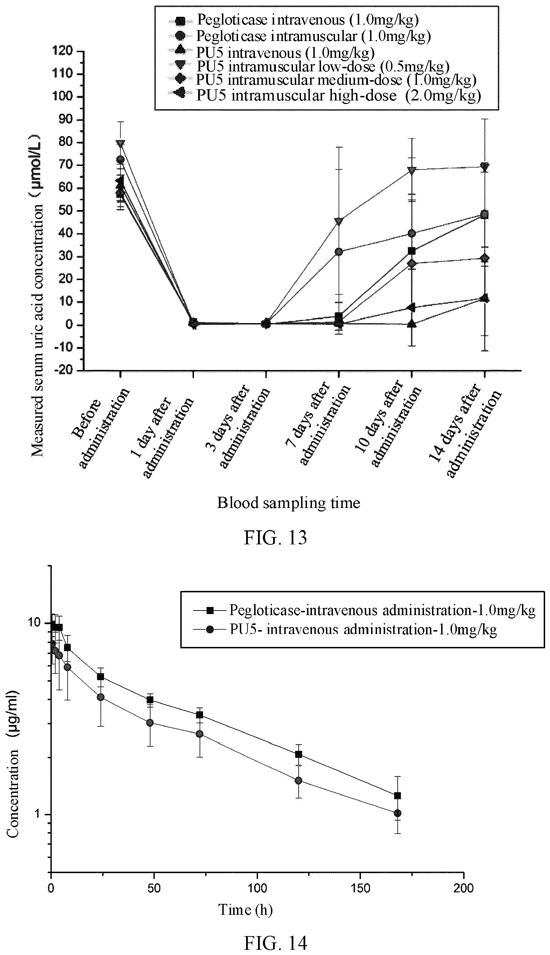

After a single intramuscular injection of 0.5 mg/kg, 1.0 mg/kg, 2.0 mg/kg pegylated uricase injection, the uric acid concentration maintained at a low level 1 day and 3 days after the administration, the uric acid level of each dose group began to recover 7 days after the administration, and the higher the dose, the longer the uric acid maintained a lower level in the body. Comparing the intravenous injection groups of the same dose, the PU5 intravenous injection group maintained a low concentration level of serum uric acid for a longer time than the pegloticase intravenous injection group. Comparing the intramuscular injection groups of the same dose, the PU5 intramuscular injection group maintained a low concentration level of serum uric acid for a longer time than the pegloticase intramuscular injection group. Comparing the groups of the same dose, the PU5 intravenous or intramuscular injection group maintained a low concentration level of serum uric acid for a longer time than the pegloticase intravenous or intramuscular injection group, that is, PU5 maintained a low concentration level of uric acid in the body for a longer time than pegloticase in each case. The results are illustrated in FIG. 13.

4.3 Evaluation of multiple administrations of polyethylene glycol-modified urate oxidase in rats

In this experiment, 4 groups were set, i.e., a marketed drug Pegloticase intravenous injection group, a Pegloticase intramuscular injection group, a pegylated uricase (PU5) intravenous injection group, and a PU5 intramuscular injection group, each group included 8 rats, four of which were male and the other four of which were female, 32 SD rats in total. The Pegloticase and pegylated uricase intravenous injection groups adopted intravenous injection; and the Pegloticase and pegylated uricase intramuscular injection groups adopted intramuscular injection. The administration dose was 1.0 mg/kg, once a week, for 4 consecutive times.

The result analysis indicates that:

SD rats were injected intravenously/intramuscularly with 1.0 mg/kg Pegloticase and pegloticase injections for multiple times, The general condition of the rats had no abnormal changes related to the drugs.

4.3.1 Anti-PEG antibody detection

SD rats were administrated for 4 consecutive times. Before the first administration, no anti-PEG antibodies and no anti-PHC antibodies were detected in either individual animal; after the administration, no anti-PHC antibodies were detected in either animal, anti-PEG antibodies were detected in each group of the Pegloticase intravenous and intramuscular injection groups as well as the pegylated uricase intravenous and intramuscular injection groups, and the ratios of positive results were: 3/8, 1/8, 1/8, and 1/8, respectively. The PEG immunohistochemical examination revealed that the spleen, liver and kidney of the pegloticase intravenous and intramuscular injection groups showed weak positive expression of PEG; and no positive expression of PEG was found in the pegylated uricase intravenous and intramuscular injection groups. The results are shown in Table 13.

From the above analysis, it can be seen that the antibodies induced by PU5 and pegloticase are mainly antibodies against the PEG part, rather than antibodies against the urate oxidase part, indicating that both can effectively mask the immunogenic sites of urate oxidase. The production of PEG antibodies may cause some side effects in the body. According to the results in Table 13, the immunogenicity of PU5 in the present disclosure is lower than that of the commercially available product pgeloticase.

According to the results of PEG antibody and PEG immunohistochemistry, the intramuscular administration groups of both PU5 and pegloticase are superior to the intravenous administration groups thereof. Among them, regarding the produced anti-PEG antibodies of the intravenous administration groups, PU5 is superior to pegloticase; and regarding the produced anti-PEG antibodies of the intramuscular administration groups, PU5 is superior to pegloticase.

[Table 13] PEG immunohistochemistry positive expression results

| | | Incidence | | | | | | | |
|---|---|---|---|---|---|---|---|---|---|
| | | Pegloticase intravenous injection group | | Pegloticase intramuscular injection group | | PU5 intravenous injection group | | PU5 intramuscular injection group | |
| Microscopic observation | | Male | Female | Male | Female | Male | Female | Male | Female |
| **Spleen** | Total inspection number: | 4 | 4 | 4 | 4 | 4 | 4 | 4 | 4 |

(continued)

| | | Incidence | | | | | | | |
|---|---|---|---|---|---|---|---|---|---|
| | | Pegloticase intravenous injection group | | Pegloticase intramuscular injection group | | PU5 intravenous injection group | | PU5 intramuscular injection group | |
| --PEG, white pulp | 1 | 0 | 1 | 1 | 2 | 0 | 0 | 0 | 0 |
| | Total incidence number incidents: | 0 | 1 | 1 | 2 | 0 | 0 | 0 | 0 |
| --PEG, red pulp | 1 | 0 | 1 | 0 | 0 | 0 | 0 | 0 | 0 |
| | Total incidence number | 0 | 1 | 0 | 0 | 0 | 0 | 0 | 0 |
| **Liver** | Total inspection number: | 4 | 4 | 4 | 4 | 4 | 4 | 4 | 4 |
| --PEG, vascular endothelial cells/Kupffer cells | 1 | 4 | 4 | 1 | 3 | 0 | 0 | 0 | 0 |
| | Total incidence number | 4 | 4 | 1 | 3 | 0 | 0 | 0 | 0 |
| **Kidney** | Total inspection number: | 4 | 4 | 4 | 4 | 4 | 4 | 4 | 4 |
| --PEG, renal tubules | 1 | 3 | 1 | 1 | 1 | 0 | 0 | 0 | 0 |
| | Total incidence number | 3 | 1 | 1 | 1 | 0 | 0 | 0 | 0 |
| --PEG, vascular endothelial cells | 1 | 0 | 1 | 0 | 0 | 0 | 0 | 0 | 0 |
| | Total incidence number | 0 | 1 | 0 | 0 | 0 | 0 | 0 | 0 |
| Positive grading: 1 = extremely weakly positive, 2 = weakly positive, 3 = moderately positive, 4 = strongly positive. | | | | | | | | | |

4.3.2 Pharmacokinetic tests

The SD rats that had received multiple intravenous and intramuscular injections of Pegloticase and pegylated uricase injections exhibited no significant gender difference in the main pharmacokinetic parameters between the groups. After 4 consecutive administrations, these two drugs accumulated slightly in rats.

When the SD rats received multiple intravenous/intramuscular injection administrations with the same dose (1.0 mg/kg) of the marketed drug Pegloticase, the absolute bioavailability in the rats was 51.35% after the first administration; and the absolute bioavailability in the rats was 45.98% after the last administration. When the SD rats received multiple intravenous/intramuscular injection administrations with the same dose (1.0 mg/kg) of the pegylated uricase injection, the absolute bioavailability in the rats was 58.29% after the first administration; and the absolute bioavailability in the rats was 52.60% after the last administration.

4.3.3 Comparison of in vivo drug efficacy (uric acid)

SD rats were injected intravenously and intramuscularly with 1.0 mg/kg Pegloticase and 1.0 mg/kg pegylated uricase injection for 4 consecutive times (1 time/week), the serum uric acid concentration maintained at a low level after each administration; the serum uric acid concentration recovered 14 days after the last administration in the Pegloticase intramuscular injection group, and in the rest groups, the serum uric acid concentration recovered 18 days after the last administration. Compared with the same dose of the marketed drug Pegloticase, the maintaining time in the intravenous injection groups of these two drugs was basically consistent, and the maintaining time of the pegylated uricase intramuscular injection group was longer than that of the marketed drug intramuscular injection group. That is, for the intramuscular administration, the efficacy of PU5 is superior than that of Pegloticase.

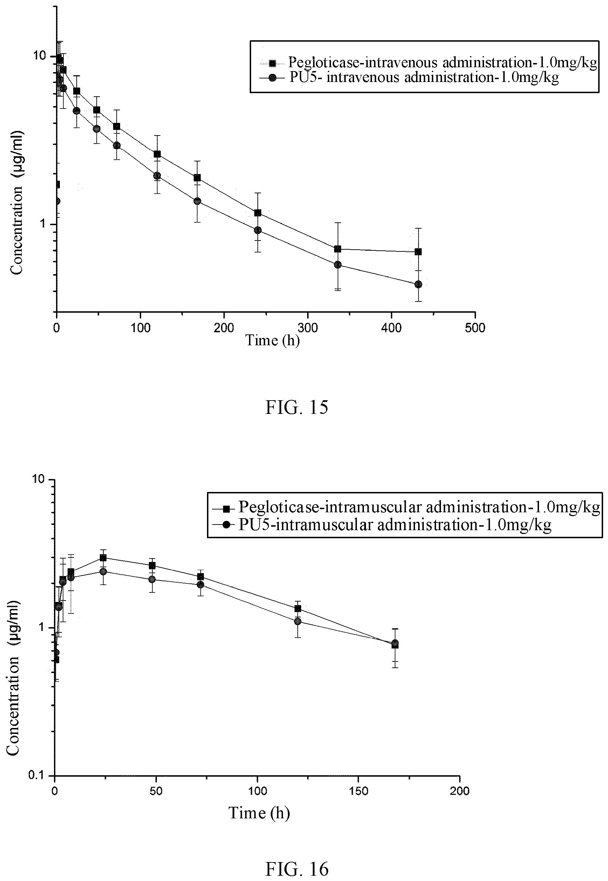

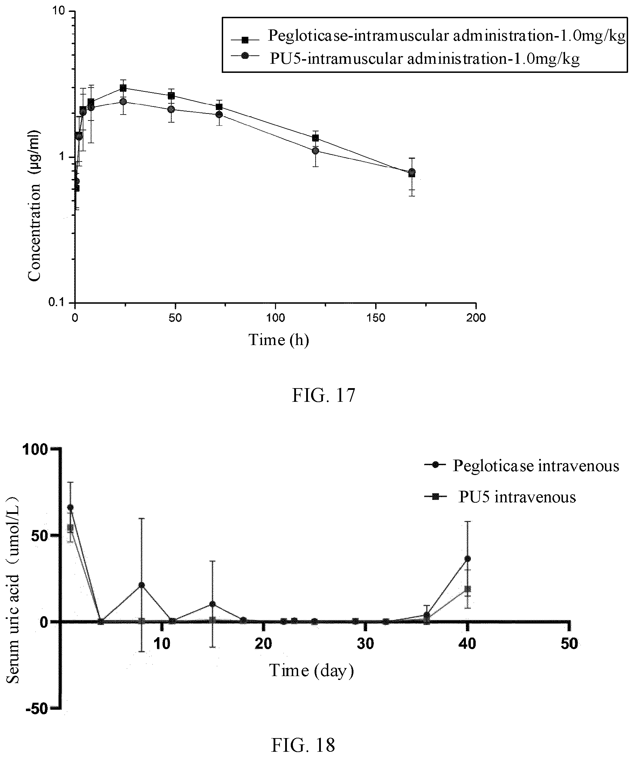

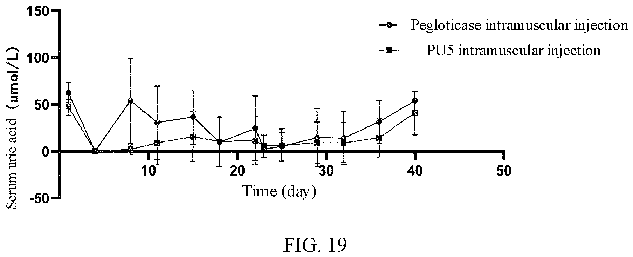

The above results are shown in Table 14 to Table 17, and FIG. 14 to FIG. 19.

[Table 14] Results of average pharmacokinetic parameters after continuous intravenous injection of Pegloticase and a pegylated uricase injection in SD rats

| Test time | Dose | Gender | Parameter | $t_{1/2}$ | $T_{max}$ | $C_{max}$ | $AUC_{last}$ | $AUC_{0-\infty}$ | Vz | Cl | $MRT_{last}$ |
|---|---|---|---|---|---|---|---|---|---|---|---|
| | Drug name | | | (h) | (h) | ($\mu$g/mL) | (h*$\mu$g/mL) | (h*$\mu$g/mL) | (mL/kg) | (mL/h/kg) | (h) |
| Day 1 | 1.0 mg/kg Pegloticase | Male | N | 4 | 4 | 4 | 4 | 4 | 4 | 4 | 4 |
| | | | Mean | 72.17 | 2.13 | 11.12 | 632.58 | 778.65 | 133.74 | 1.29 | 56.91 |
| | | | SD | 4.53 | 1.44 | 0.28 | 25.32 | 45.00 | 4.49 | 0.07 | 1.17 |
| | | Female | N | 4 | 4 | 4 | 4 | 4 | 4 | 4 | 4 |
| | | | Mean | 66.24 | 0.50 | 8.84 | 514.21 | 633.80 | 149.07 | 1.59 | 54.93 |
| | | | SD | 17.91 | 0.00 | 1.07 | 50.98 | 64.84 | 27.23 | 0.16 | 7.58 |
| | | Female+Male | N | 8 | 8 | 8 | 8 | 8 | 8 | 8 | 8 |
| | | | Mean | 69.21 | 1.31 | 9.98 | 573.40 | 706.22 | 141.41 | 1.44 | 55.92 |
| | | | SD | 12.51 | 1.28 | 1.42 | 73.43 | 93.08 | 19.84 | 0.20 | 5.13 |
| | 1.0 mg/kg PU5 | Male | N | 4 | 4 | 4 | 4 | 4 | 4 | 4 | 4 |
| | | | Mean | 67.98 | 1.75 | 9.47 | 527.73 | 634.48 | 158.66 | 1.60 | 55.50 |
| | | | SD | 8.87 | 1.66 | 0.91 | 91.07 | 91.12 | 39.53 | 0.22 | 0.89 |
| | | Female | N | 4 | 4 | 4 | 4 | 4 | 4 | 4 | 4 |
| | | | Mean | 79.29 | 1.38 | 6.42 | 369.05 | 481.09 | 238.12 | 2.16 | 59.68 |
| | | | SD | 17.07 | 1.75 | 0.61 | 49.11 | 98.31 | 15.34 | 0.53 | 2.84 |
| | | Female+Male | N | 8 | 8 | 8 | 8 | 8 | 8 | 8 | 8 |
| | | | Mean | 73.63 | 1.56 | 7.94 | 448.39 | 557.79 | 198.39 | 1.88 | 57.59 |
| | | | SD | 13.97 | 1.59 | 1.78 | 108.54 | 120.10 | 50.74 | 0.48 | 2.96 |

| Test time | Dose | Gender | Parameter | $t_{1/2}$ | $T_{max}$ | $C_{max}$ | $AUC_{last}$ | $AUC_{0-\infty}$ | Vz | Cl | $MRT_{last}$ |
| | Drug name | | | (h) | (h) | (μg/mL) | (h*μg/mL) | (h*μg/mL) | (mL/kg) | (mL/h/kg) | (h) |
|---|---|---|---|---|---|---|---|---|---|---|---|
| Day 22 | 1.0 mg/kg Pegloticase | Male | N | 4 | 4 | 4 | 4 | 4 | 4 | 4 | 4 |
| | | | Mean | 135.63 | 1.75 | 12.33 | 1159.18 | 1300.99 | 149.28 | 0.78 | 118.29 |
| | | | SD | 40.45 | 1.66 | 0.94 | 134.20 | 208.65 | 28.13 | 0.13 | 9.91 |
| | | Female | N | 4 | 4 | 4 | 4 | 4 | 4 | 4 | 4 |
| | | | Mean | 96.09 | 0.88 | 8.02 | 672.95 | 747.61 | 186.89 | 1.35 | 92.46 |
| | | | SD | 7.69 | 0.75 | 0.87 | 78.50 | 78.66 | 24.21 | 0.14 | 9.52 |
| | | Female+Male | N | 8 | 8 | 8 | 8 | 8 | 8 | 8 | 8 |
| | | | Mean | 115.86 | 1.31 | 10.17 | 916.07 | 1024.30 | 168.09 | 1.07 | 105.37 |
| | | | SD | 34.25 | 1.28 | 2.45 | 279.12 | 329.86 | 31.53 | 0.33 | 16.48 |
| | 1.0 mg/kg PU5 | Male | N | 4 | 4 | 4 | 4 | 4 | 4 | 4 | 4 |
| | | | Mean | 149.80 | 2.25 | 9.00 | 840.78 | 947.08 | 229.16 | 1.07 | 117.99 |
| | | | SD | 24.68 | 2.02 | 0.73 | 91.96 | 104.82 | 36.69 | 0.11 | 6.88 |
| | | Female | N | 4 | 4 | 4 | 4 | 4 | 4 | 4 | 4 |
| | | | Mean | 103.90 | 1.25 | 6.63 | 576.81 | 636.52 | 236.33 | 1.63 | 101.66 |
| | | | SD | 21.25 | 0.87 | 0.72 | 128.81 | 128.02 | 17.98 | 0.39 | 20.03 |
| | | Female+Male | N | 8 | 8 | 8 | 8 | 8 | 8 | 8 | 8 |
| | | | Mean | 126.85 | 1.75 | 7.82 | 708.79 | 791.80 | 232.75 | 1.35 | 109.82 |
| | | | SD | 32.51 | 1.54 | 1.44 | 175.05 | 198.21 | 27.02 | 0.40 | 16.38 |

[Table 15] Results of average pharmacokinetic parameters after continuous intramuscular injection of Pegloticase and pegylated uricase injection in SD rats

| Test time | Dose | Gender | Parameter | $t_{1/2}$ | $T_{max}$ | $C_{max}$ | $AUC_{last}$ | $AUC_{0-\infty}$ | Vz_F | Cl_F | $MRT_{last}$ |
|---|---|---|---|---|---|---|---|---|---|---|---|
| | Drug name | | | (h) | (h) | ($\mu$g/mL) | (h*$\mu$g/mL) | (h*$\mu$g/mL) | (mL/kg) | (mL/h/kg) | (h) |
| Day 1 | 1.0 mg/kg Pegloticase | Male | N | 4 | 4 | 4 | 4 | 4 | 4 | 4 | 4 |
| | | | Mean | 59.68 | 24.00 | 3.16 | 318.22 | 395.66 | 217.53 | 2.54 | 62.05 |
| | | | SD | 13.70 | 0.00 | 0.38 | 29.92 | 29.26 | 49.49 | 0.19 | 6.59 |
| | | Female | N | 4 | 4 | 4 | 4 | 4 | 4 | 4 | 4 |
| | | | Mean | 80.53 | 24.00 | 2.80 | 270.69 | 415.60 | 282.07 | 2.48 | 57.80 |
| | | | SD | 16.48 | 0.00 | 0.36 | 66.91 | 84.31 | 37.74 | 0.52 | 6.21 |
| | | Female+Male | N | 8 | 8 | 8 | 8 | 8 | 8 | 8 | 8 |
| | | | Mean | 70.11 | 24.00 | 2.98 | 294.46 | 405.63 | 249.80 | 2.51 | 59.92 |
| | | | SD | 17.91 | 0.00 | 0.39 | 54.29 | 59.39 | 53.39 | 0.36 | 6.35 |
| | 1.0 mg/kg PU5 | Male | N | 4 | 4 | 4 | 4 | 4 | 4 | 4 | 4 |
| | | | Mean | 82.25 | 6.00 | 3.06 | 290.64 | 403.89 | 294.92 | 2.50 | 61.83 |
| | | | SD | 9.79 | 2.31 | 0.25 | 34.67 | 48.73 | 29.13 | 0.31 | 6.88 |
| | | Female | N | 4 | 4 | 4 | 4 | 4 | 4 | 4 | 4 |
| | | | Mean | 70.44 | 48.00 | 2.21 | 232.11 | 306.59 | 340.32 | 3.50 | 66.28 |
| | | | SD | 13.41 | 19.60 | 0.26 | 59.53 | 90.62 | 46.97 | 1.09 | 10.28 |
| | | Female+Male | N | 8 | 8 | 8 | 8 | 8 | 8 | 8 | 8 |
| | | | Mean | 76.34 | 27.00 | 2.63 | 261.38 | 355.24 | 317.62 | 3.00 | 64.06 |
| | | | SD | 12.57 | 25.90 | 0.51 | 54.89 | 85.10 | 43.56 | 0.91 | 8.44 |

| Test time | Dose | Gender | Parameter | $t_{1/2}$ | $T_{max}$ | $C_{max}$ | $AUC_{last}$ | $AUC_{0-\infty}$ | Vz_F | Cl_F | $MRT_{last}$ |
|---|---|---|---|---|---|---|---|---|---|---|---|
| | Drug name | | | (h) | (h) | (μg/mL) | (h*μg/mL) | (h*μg/mL) | (mL/kg) | (mL/h/kg) | (h) |
| Day 22 | 1.0 mg/kg Pegloticase | Male | N | 3 | 4 | 4 | 4 | 3 | 3 | 3 | 4 |
| | | | Mean | 198.20 | 25.00 | 3.18 | 486.70 | 799.90 | 353.52 | 1.35 | 112.01 |
| | | | SD | 83.85 | 18.00 | 0.85 | 298.21 | 293.71 | 23.56 | 0.42 | 60.30 |
| | | Female | N | 4 | 4 | 4 | 4 | 4 | 4 | 4 | 4 |
| | | | Mean | 97.92 | 20.00 | 2.69 | 355.77 | 427.75 | 344.97 | 2.65 | 94.51 |
| | | | SD | 33.98 | 8.00 | 0.71 | 134.18 | 155.69 | 88.41 | 1.19 | 30.45 |
| | | Female+Male | N | 7 | 8 | 8 | 8 | 7 | 7 | 7 | 8 |
| | | | Mean | 140.90 | 22.50 | 2.93 | 421.23 | 587.25 | 348.64 | 2.09 | 103.26 |
| | | | SD | 76.12 | 13.17 | 0.77 | 225.23 | 283.63 | 64.14 | 1.12 | 45.20 |
| | 1.0 mg/kg PU5 | Male | N | 4 | 4 | 4 | 4 | 4 | 4 | 4 | 4 |
| | | | Mean | 140.04 | 15.00 | 2.52 | 395.82 | 478.83 | 610.95 | 9.64 | 102.76 |
| | | | SD | 90.61 | 10.52 | 1.15 | 255.45 | 300.26 | 419.13 | 16.09 | 61.50 |
| | | Female | N | 4 | 4 | 4 | 4 | 4 | 4 | 4 | 4 |
| | | | Mean | 122.51 | 30.00 | 2.41 | 349.84 | 428.61 | 416.41 | 2.82 | 103.39 |
| | | | SD | 59.33 | 12.00 | 0.50 | 178.08 | 204.30 | 72.32 | 1.36 | 44.91 |
| | | Female+Male | N | 8 | 8 | 8 | 8 | 8 | 8 | 8 | 8 |
| | | | Mean | 131.27 | 22.50 | 2.46 | 372.83 | 453.72 | 513.68 | 6.23 | 103.08 |
| | | | SD | 71.52 | 13.17 | 0.82 | 205.33 | 239.26 | 297.23 | 11.18 | 49.85 |

EP 3 967 754 A1

[Table 16] Statistical results of uric acid in each dose group after multiple intramuscular/intravenous injections of Pegloticase and a pegylated uricase injection in SD rats (Mean + SD)

| Gender Detection Time | 1.0 mg/kg Pegloticase intravenous injection group | | 1.0 mg/kg Pegloticas intramuscular injection group | | 1.0 mg/kg PU5 intravenous injection group | | 1.0 mg/kg PU5 intramuscular injection group | |
|---|---|---|---|---|---|---|---|---|
| | n | $\overline{X} \pm SD$ | n | $\overline{X} \pm SD$ | n | $\overline{X} \pm SD$ | n | $\overline{X} \pm SD$ |
| Male | | | | | | | | |
| After 1st administration | 4 | 71.250± 19.103 | 4 | 62.250± 5.315 | 4 | 58.750± 7.632 | 4 | 50.750± 6.850 |
| 3 days after 1st administration | 4 | 0.250± 0.500 | 4 | 0.500± 0.577 | 4 | 0± 0 | 4 | 0± 0 |
| Before 2nd administration | 4 | 0.500± 0.577 | 4 | 69.750± 46.133 | 4 | 0.500± 0.577 | 4 | 0.250± 0.500 |
| 3 days after 2nd administration | 4 | 1.000± 0 | 4 | 20.750± 40.178 | 4 | 0.500± 0.577 | 4 | 17.500± 33.670 |
| Before 3rd administration | 4 | 1.000± 0 | 4 | 26.500± 30.116 | 4 | 1.250± 0.957 | 4 | 18.000± 32.680 |
| 3 days after 3rd administration | 4 | 1.000± 0.816 | 4 | 19.000± 37.336 | 4 | 0.500± 0.577 | 4 | 20.500± 38.336 |
| Before 4th administration | 4 | 0± 0 | 4 | 15.500± 31.000 | 4 | 0.250± 0.500 | 4 | 19.250± 37.170 |
| 1 day after 4th administration | 4 | 0.750± 0.500 | 4 | 1.750± 1.500 | 4 | 0.750± 0.500 | 4 | 9.750± 16.840 |
| 3 days after 4th administration | 4 | 0.500± 0.577 | 4 | 10.500± 21.000 | 4 | 0± 0 | 4 | 12.750± 24.838 |
| 7 days after 4th administration | 4 | 0.250± 0.500 | 4 | 22.250± 44.500 | 4 | 0± 0 | 4 | 16.250± 31.837 |
| 10 days after 4th administration | 4 | 0± 0 | 4 | 20.000± 38.670 | 4 | 0.250± 0.500 | 4 | 16.000± 30.681 |
| 14 days after 4th administration | 4 | 1.250± 0.500 | 4 | 29.250± 28.123 | 4 | 2.000± 0.816 | 4 | 19.250± 30.015 |
| 18 days after 4th administration | 4 | 25.000± 10.296 | 4 | 53.500± 14.933 | 4 | 24.000± 13.614 | 4 | 50.000± 29.833 |
| Female | | | | | | | | |
| Before 1st administration | 4 | 61.250± 8.057 | 4 | 63.000± 15.470 | 4 | 50.250± 7.500 | 4 | 43.250± 9.743 |
| 3 days after 1st administration | 4 | 0± 0 | 4 | 0± 0 | 4 | 0.250± 0.500 | 4 | 0± 0 |
| Before 2nd administration | 4 | 42.000± 48.132 | 4 | 38.250± 44.507 | 4 | 0.500± 0.577 | 4 | 4.000± 7.348 |
| 3 days after 2nd administration | 4 | 0.250± 0.500 | 4 | 40.250± 41.080 | 4 | 0.250± 0.500 | 4 | 0.500± 0.577 |
| Before 3rd administration | 4 | 19.500± 35.01 | 4 | 46.750± 28.547 | 4 | 1.000± 0.816 | 4 | 13.500± 25.000 |

(continued)

| Gender Detection Time | 1.0 mg/kg Pegloticase intravenous injection group | | 1.0 mg/kg Pegloticas intramuscular injection group | | 1.0 mg/kg PU5 intravenous injection group | | 1.0 mg/kg PU5 intramuscular injection group | |
|---|---|---|---|---|---|---|---|---|
| | n | $\overline{X} \pm SD$ | n | $\overline{X} \pm SD$ | n | $\overline{X} \pm SD$ | n | $\overline{X} \pm SD$ |
| 3 days after 3rd administration | 4 | 1.250±0.500 | 4 | 0.500±0.577 | 4 | 0.750±0.500 | 4 | 0.750±0.500 |
| Before 4th administration | 4 | 0.250±0.500 | 4 | 33.750±40.285 | 4 | 0.500±0.577 | 4 | 3.750±6.850 |
| 1 day after 4th administration | 4 | 0.750±0.500 | 4 | 3.000±2.708 | 4 | 0.500±0.577 | 4 | 1.250±0.500 |
| 3 days after 4th administration | 4 | 0.250±0.500 | 4 | 0±0 | 4 | 0±0 | 4 | 0±0 |
| 7 days after 4th administration | 4 | 0.250±0.500 | 4 | 6.750±12.842 | 4 | 1.000±0 | 4 | 1.750±1.258 |
| 10 days after 4th administration | 4 | 0±0 | 4 | 8.500±16.340 | 4 | 0.250±0.500 | 4 | 2.000±2.828 |
| 14 days after 4th administration | 4 | 6.750±7.089 | 4 | 33.500±19.689 | 4 | 1.500±0.577 | 4 | 9.500±8.699 |
| 18 days after 4th administration | 4 | 48.000±24.993 | 4 | 54.750±4.031 | 4 | 14.000±6.00. | 4 | 32.000±13.638 |

In the specification, descriptions with reference to the terms "an embodiment", "some embodiments", "examples", "specific examples", or "some examples", etc., mean specific features, structures, materials or characteristics described in conjunction with the embodiment or example are included in at least one embodiment or example of the present disclosure. In this specification, the above terms are illustrative, and do not necessarily refer to the same embodiment or example. Moreover, the described specific features, structures, materials or characteristics can be combined in a suitable manner in any one or more embodiments or examples. In addition, those skilled in the art can combine the different embodiments or examples and the features of the different embodiments or examples described in this specification without contradicting each other.

Although the embodiments of the present disclosure are illustrated and described above, it can be understood that the above-mentioned embodiments are illustrative and should not be construed as limitations of the present disclosure. Those skilled in the art can make changes, modifications, substitutions, and variations based on the above-mentioned embodiments within the scope of the present disclosure.

## Claims

1. A polyethylene glycol-modified urate oxidase, wherein at least 11 of the following amino acid sites in the urate oxidase have a PEG modification:
$T^1$, $K^3$, $K^4$, $K^{30}$, $K^{35}$, $K^{76}$, $K^{79}$, $K^{97}$, $K^{112}$, $K^{116}$, $K^{120}$, $K^{152}$, $K^{179}$, $K^{222}$, $K^{231}$, $K^{266}$, $K^{272}$, $K^{285}$, $K^{291}$, and $K^{293}$.

2. The urate oxidase according to claim 1, wherein at least one, at least two, at least three, or four of the following 4 amino acid sites in the urate oxidase have the PEG modification:
$K^{30}$, $K^{35}$, $K^{222}$, and $K^{231}$.

3. The urate oxidase according to claim 1, wherein polyethylene glycol used for the PEG modification has a molecular weight smaller than or equal to 6KD.

4. The urate oxidase according to claim 1, wherein the polyethylene glycol has a monomethoxyl group or a hydroxyl group;

> optionally, the polyethylene glycol is a linear or branched structure;
> optionally, the polyethylene glycol and the urate oxidase are coupled through an amide bond;
> preferably, the polyethylene glycol is a modifying polyethylene glycol, and a modifying group of the modifying polyethylene glycol comprises at least one selected from the group consisting of: N-hydroxysuccinimide, a N-hydroxysuccinimidyl carbonate ester, a N-hydroxysuccinimidyl acetate ester, a N-hydroxysuccinimidyl propionate ester, a N-hydroxysuccinimidyl butyrate ester, a N-hydroxysuccinyl succinate ester, and a bis(4-nitrophenyl) carbonate ester; and
> preferably, the modifying group of the modifying polyethylene glycol is the N-hydroxysuccinimidyl propionate ester.

5. The urate oxidase according to claim 1, wherein the amino acid sites are positioned based on an amino acid sequence set forth as SEQ ID NO: 1;

> optionally, the urate oxidase has amino acid sequences set forth as SEQ ID NO: 1 to SEQ ID NO: 7; or
> the urate oxidase has a polypeptide having at least 70%, at least 75%, at least 80%, at least 85%, at least 90%, at least 95%, or at least 99% identity to SEQ ID NO: 1 to SEQ ID NO: 7; or
> the urate oxidase has a polypeptide having the amino acid sequences set forth as SEQ ID NO: 1 to SEQ ID NO: 7 in which one or more amino acids are substituted, deleted and/or added; and
> preferably, the urate oxidase has amino acid sequences set forth as SEQ ID NO: 1 to SEQ ID NO: 4.

6. A polyethylene glycol-modified urate oxidase, wherein peak areas of at least 11 predetermined peptide fragments in a peptide map of the polyethylene glycol-modified urate oxidase are reduced by a relative proportion of 75% or more, preferably 80% or more, more preferably 90% or more, compared with those in a peptide map of urate oxidase unmodified with polyethylene glycol.

7. The polyethylene glycol-modified urate oxidase according to claim 6, wherein the peptide map of the polyethylene glycol-modified urate oxidase has peptide fragments with peak area reduction shown in Table 5.

8. The polyethylene glycol-modified urate oxidase according to claim 6, wherein the peptide map of the polyethylene glycol-modified urate oxidase is shown in FIG. 6 or FIG. 7.

9. A pharmaceutical composition, comprising the urate oxidase according to any one of claims 1 to 8.

10. The pharmaceutical composition according to claim 9, further comprising an additional drug for treatment or prevention of hyperuric acid-related diseases through drug combination.

11. Use of the urate oxidase according to any one of claims 1 to 8, or the pharmaceutical composition according to any one of claims 9 to 10 in manufacture of a medicament for treating hyperuric acid-related diseases and reducing a uric acid level in a biological fluid of a subject in need thereof, wherein

> optionally, the hyperuric acid-related diseases comprise chronic hyperuricemia, gout, kidney disease, hyperuricemic arthritis, renal calculi, tophus, hypertension, diabetes, hypertriglyceridemia, mtabolic syndrome, coronary heart disease, atherosclerosis, and cancer chemotherapy-induced hyperuricemia; and
> optionally, the biological fluid is urine or blood.

12. A method for reducing immunogenicity of urate oxidase, comprising:
enabling at least 11 of the following amino acid sites in the urate oxidase to have a PEG modification:
$T^1$, $K^3$, $K^4$, $K^{30}$, $K^{35}$, $K^{76}$, $K^{79}$, $K^{97}$, $K^{112}$, $K^{116}$, $K^{120}$, $K^{152}$, $K^{179}$, $K^{222}$, $K^{231}$, $K^{266}$, $K^{272}$, $K^{285}$, $K^{291}$, and $K^{293}$.

13. The method according to claim 12, comprising:
enabling at least one, at least two, at least three, or four of the following 4 amino acid sites in the urate oxidase to have a PEG modification:
$K^{30}$, $K^{35}$, $K^{222}$, and $K^{231}$.

14. The method according to claim 12, wherein polyethylene glycol used for the PEG modification has a molecular weight smaller than or equal to 6KD;

preferably, the polyethylene glycol is a modifying polyethylene glycol; and
preferably, a modifying group of the modifying polyethylene glycol is N-hydroxysuccinimide.

15. The method according to claim 12, wherein the amino acid sites are positioned based on an amino acid sequence set forth as SEQ ID NO: 1;

preferably, the urate oxidase has amino acid sequences set forth as SEQ ID NO: 1 to SEQ ID NO: 7; or
the urate oxidase has a polypeptide having at least 70%, at least 75%, at least 80%, at least 85%, at least 90%, at least 95%, or at least 99% identity to SEQ ID NO: 1 to SEQ ID NO: 7; or
the urate oxidase has a polypeptide having the amino acid sequences set forth as SEQ ID NO: 1 to SEQ ID NO: 7 in which one or more amino acids are substituted, deleted and/or added; and
preferably, the urate oxidase has amino acid sequences set forth as SEQ ID NO: 1 to SEQ ID NO: 4.

16. A method for treating or preventing hyperuric acid-related diseases and reducing a uric acid level in a biological fluid of a subject in need thereof, comprising:
administering a therapeutically effective amount of the urate oxidase according to any one of claims 1 to 8 or the pharmaceutical composition according to any one of claims 9 to 10 to the subject.

17. The method according to claim 16, wherein the hyperuric acid-related diseases comprise chronic hyperuricemia, gout, kidney disease, hyperuricemic arthritis, renal calculi, tophus, hypertension, diabetes, hypertriglyceridemia, mtabolic syndrome, coronary heart disease, atherosclerosis, and cancer chemotherapy-induced hyperuricemia; and optionally, the biological fluid is urine or blood.

FIG. 1

FIG. 2

FIG. 3

FIG. 4

FIG. 5

FIG. 6

FIG. 7

G1: Blank control group (n=10)
G2: Model control group (n=10)
G3: PU5 low-dose group (n=10)
G4: PU5 medium-dose group (n=10)
G5: PU5 high-dose group (n=10)

FIG. 8

FIG. 9

FIG. 10

FIG. 11

FIG. 12

FIG. 13

FIG. 14

FIG. 15

FIG. 16

FIG. 17

FIG. 18

FIG. 19

<div align="center">

**INTERNATIONAL SEARCH REPORT**

</div>

| | International application No. |
|---|---|
| | **PCT/CN2020/089272** |

| A. | CLASSIFICATION OF SUBJECT MATTER |
|---|---|

C12N 9/06(2006.01)i; A61K 38/44(2006.01)i; A61K 47/56(2017.01)i; A61P 19/02(2006.01)i; A61P 19/06(2006.01)i; A61P 13/12(2006.01)i

According to International Patent Classification (IPC) or to both national classification and IPC

| B. | FIELDS SEARCHED |
|---|---|

Minimum documentation searched (classification system followed by classification symbols)

C12N; A61K; A61P

Documentation searched other than minimum documentation to the extent that such documents are included in the fields searched

Electronic data base consulted during the international search (name of data base and, where practicable, search terms used)

VEN; TWTXT; GBTXT; SGTXT; EPTXT; USTXT; CHTXT; WOTXT; CATXT; CNTXT; ISI WEB OF SCIENCES; PUBMED; CNKI; 万方: 聚乙二醇, PEG, 尿酸氧化酶, 尿酸酶, 赖氨酸, Lys, 痛风, 高尿酸血症, 免疫原性, 稳定性, polyethylene glycol, urate oxidase, uricase, UOX, lysine, gout, hyperuricemia, immunogenicity, stability

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | CN 102634492 A (CHONGQING FUJIN BIO-PHARM CO., LTD.) 15 August 2012 (2012-08-15) claims 1-9, description paragraphs 5, 20 | 1-17 |
| Y | 李晶 等 (LI, Jing et al.). "液相肽图法推断聚乙二醇化重组人生长激素的聚乙二醇修饰位点研究 (RP-HPLC Peptide Mapping to Determine Pegylation Sites of PEG-rhGH)" 中国药学杂志 (Chinese Pharmaceutical Journal), Vol. 47, No. 8, 30 April 2012 (2012-04-30), pp. 626-630 | 1-17 |
| Y | 张平 (ZHANG, Ping). "YPEG-rhIFNα2a修饰位点结构解析 (Non-official translation: Analysis on the Structure of YPEG-rhIFN α2a Modification Sites)" 海峡药学 (Strait Pharmaceutical Journal), Vol. 31, No. 2,, 28 February 2019 (2019-02-28), pp. 58-62 | 1-17 |
| A | WO 2003011211 A2 (PHOENIX PHAMACOLOGICS, INC.) 13 February 2003 (2003-02-13) entire document | 1-17 |
| A | JP 6-319537 A (DAIKIN IND., LTD) 22 November 1994 (1994-11-22) entire document | 1-17 |

☑ Further documents are listed in the continuation of Box C.     ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **09 July 2020** | **13 August 2020** |

| Name and mailing address of the ISA/CN | Authorized officer |
|---|---|
| **China National Intellectual Property Administration (ISA/ CN)** **No. 6, Xitucheng Road, Jimenqiao Haidian District, Beijing 100088** **China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2020/089272**

**C.    DOCUMENTS CONSIDERED TO BE RELEVANT**

| Category* | Citation of document, with indication, where appropriate, of the relevant passages | Relevant to claim No. |
|---|---|---|
| A | CN 109682901 A (CHONGQING PEG-BIO BIOTECHNOLOGY CO., LTD.) 26 April 2019 (2019-04-26)<br>     entire document | 1-17 |
| A | NANDA P. et al. "Production and Optimization of Site-Specific monoPEGylated Uricase Conjugates Using mPEG-Maleimide Through RP–HPLC Methodology"<br>*J. Pharm. Innov.*, Vol. 11,, 10 June 2016 (2016-06-10),<br>     pp. 279-288 | 1-17 |
| A | SHERMAN M. R. et al. "PEG-uricase in the management of treatment-resistant gout and hyperuricemia"<br>*Advanced Drug Delivery Reviews*, Vol. 60, 14 August 2007 (2007-08-14),<br>     pp. 59-68 | 1-17 |

Form PCT/ISA/210 (second sheet) (January 2015)

**EP 3 967 754 A1**

## INTERNATIONAL SEARCH REPORT

| International application No. |
| --- |
| **PCT/CN2020/089272** |

| Box No. II | Observations where certain claims were found unsearchable (Continuation of item 2 of first sheet) |
| --- | --- |

This international search report has not been established in respect of certain claims under Article 17(2)(a) for the following reasons:

1. ☑ Claims Nos.: **16-17**
because they relate to subject matter not required to be searched by this Authority, namely:

    [1]   Claims 16-17 relate to a high uric acid-related disease treatment method, which does not satisfy the criteria of PCT Rule 39.1(iv). The present report takes a corresponding pharmaceutical use invention as the basis for performing a search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

62

**INTERNATIONAL SEARCH REPORT**
Information on patent family members

International application No.

**PCT/CN2020/089272**

| Patent document cited in search report | | | Publication date (day/month/year) | Patent family member(s) | | | Publication date (day/month/year) |
|---|---|---|---|---|---|---|---|
| CN | 102634492 | A | 15 August 2012 | EP | 2684950 | A4 | 07 January 2015 |
| | | | | US | 9193967 | B2 | 24 November 2015 |
| | | | | WO | 2012109975 | A1 | 23 August 2012 |
| | | | | CN | 102634492 | B | 10 June 2015 |
| | | | | EP | 2684950 | A1 | 15 January 2014 |
| | | | | US | 2014065123 | A1 | 06 March 2014 |
| WO | 2003011211 | A2 | 13 February 2003 | None | | | |
| JP | 特开平6-319537 | A | 22 November 1994 | JP | H06319537 | A | 22 November 1994 |
| CN | 109682901 | A | 26 April 2019 | None | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7056713 B1, Michael H, Susan J.K.
- CN 1264575 C

**Non-patent literature cited in the description**

- **WU X ; LEE C C ; MUZNY D M ; CASKEY C T.** *Proc Natl Acad Sci USA.,* 1989, vol. 86, 9412-9416
- **WU X ; MUZNY D M ; LEE C ; CASKEY C T.** *J Mol Evol,* 1992, vol. 34, 78-84
- **WORTMANN R L ; KELLEY W N.** Kelley's textbook of rheumatology. 2001, 1339-1376
- **ZITTOUN R ; DAUCHY F ; TEILAUD C ; BARTHELEMY M ; BOUCHARD P.** *Ann Med Interne,* 1978, vol. 127, 479-482
- **PUI C H ; RELLING M V ; LASCOMBES F ; HARRISONP L ; STRUXIANO A et al.** *Leukemia,* 1997, vol. 11, 1813-1816
- **POTAUX L ; APARICIO M ; MAUREL C ; RUEDAS M E ; MART IN C L.** *Nouv PresseMed,* 1975, vol. 4, 1109-1112
- **SUZUKI K ; SAKASEGAWA S ; MISAKI H ; SUGIYAMA M.** *J Biosci Bioeng,* 2004, vol. 98, 153-158
- **RETAILLEAU P ; COLLOC'H ; DENIS V ; FRANCOISE B.** *Acta Cryst D,* 2004, vol. 60, 453-462
- **BAYOL A et al.** *Biophys Chem,* 1995, vol. 54, 229-235
- **HUANG S H ; WU T K.** *Eur J Biochem,* 2004, vol. 271, 517-523
- **LEE C C ; WU X ; GIBBS R A ; COOK R G ; MUZNY D M ; CASKEYC T.** *Science,* 1988, vol. 239, 1288-1291